(19) Europäisches Patentamt
European Patent Office
Office européen des brevets

(11) Veröffentlichungsnummer: **0 111 455**
**A2**

(12) # EUROPÄISCHE PATENTANMELDUNG

(21) Anmeldenummer: 83810575.7

(22) Anmeldetag: 08.12.83

(51) Int. Cl.³: C 07 D 491/048
A 61 K 31/435
//(C07D491/048, 307/00, 221/00)

(30) Priorität: 10.12.82 CH 7212/82
25.03.83 CH 1639/83

(43) Veröffentlichungstag der Anmeldung: 20.06.84 Patentblatt 84/25

(84) Benannte Vertragsstaaten: AT BE CH DE FR GB IT LI NL SE

(71) Anmelder: CIBA-GEIGY AG
Postfach
CH-4002 Basel(CH)

(72) Erfinder: Kühnis, Hans, Dr.
Lange Gasse 26
CH-4052 Basel(CH)

(54) **Ungesättigte Lactone.**

(57) Die Erfindung betrifft neue 5-Oxo-1,4,5,7-tetrahydrofuro [3,4-b]-pyridin-3-verbindungen der Formel

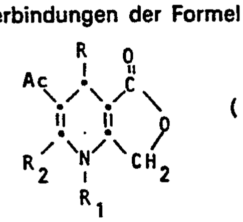

(I)

worin R einen durch Niederalkoxyniederalkoxy, Halogenniederalkoxy, ausgenommen Trifluormethoxy, Niederalkylthioniederalkoxy oder Halogenniederalkylthio substituierten carbocyclischen oder heterocyclischen Arylrest bedeutet, $R_1$ Wasserstoff oder unsubstituiertes oder substituiertes Niederalkyl bedeutet, $R_2$ Wasserstoff, Niederalkyl oder durch freies, veräthertes oder verestertes Hydroxy, durch funktionell abgewandeltes Carboxy oder durch freies oder substituiertes Amino substituiertes Niederalkyl, funktionell abgewandeltes Carboxy oder freies oder substituiertes Amino bedeutet, wobei eine Aminogruppe $R_2$ mit einem Niederalkylrest $R_1$ verbunden sein kann, oder, falls $R_2$ für Niederalkyl steht, dieses zusammen mit dem benachbarten Acylrest Ac einen 2-Oxa-1-oxo-niederalkylenrest bilden kann, wobei dessen Carbonylgruppe mit dem 3-Ringkohlenstoffatom des Furo [3,4-b] pyridinrings verbunden ist, und Ac den Acylrest einer Säure darstellt, sowie von pharmazeutisch verwendbaren Salzen von Verbindungen der Formel I mit salzbildenden Eigenschaften, als cardiotonisch wirksame und blutdrucksteigernde, sowie blutzuckersenkende, ferner das Zentralnervensystem aktivierende, insbesondere stimulierende, Verbindungen, pharmazeutische Präparate enthaltend solche Verbindungen sowie Verfahren zu deren Herstellung.

EP 0 111 455 A2

CIBA-GEIGY AG 4-14218/1+2/-/+

Basel (Schweiz)

## Ungesättigte Lactone

Die Erfindung betrifft neue ungesättigte Lacton-Verbindungen, Verfahren zu ihrer Herstellung, pharmazeutische Präparate enthaltend solche Verbindungen, und ihre Verwendung zur Herstellung von pharmazeutischen Präparaten, oder als pharmakologisch aktive Verbindungen.

Die Erfindung betrifft in erster Linie neue 5-Oxo-1,4,5,7-tetrahydrofuro[3,4-b]pyridin-3-verbindungen der Formel

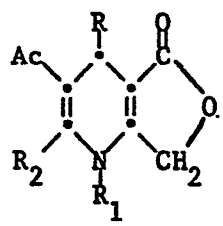

(I)

worin R einen durch Niederalkoxyniederalkoxy, Halogenniederalkoxy ausgenommen Trifluormethoxy, Niederalkylthioniederalkoxy oder Halogenniederalkylthio substituierten carbocyclischen oder heterocyclischen Arylrest bedeutet, $R_1$ Wasserstoff oder unsubstituiertes oder substituiertes Niederalkyl bedeutet, $R_2$ Wasserstoff, Niederalkyl oder durch freies, veräthertes oder verestertes Hydroxy, durch funktionell abgewandeltes Carboxy oder durch freies oder substituiertes Amino substituiertes Niederalkyl, funktionell abgewandeltes Carboxy oder freies oder substituiertes Amino bedeutet, wobei eine Aminogruppe $R_2$ mit einem Niederalkylrest $R_1$ verbunden sein kann, oder, falls $R_2$ für Niederalkyl steht, dieses zusammen mit dem benachbarten Acylrest Ac einen 2-Oxa-1-oxo-niederalkylenrest bilden

kann, wobei dessen Carbonylgruppe mit dem 3-Ringkohlenstoffatom des Furo[3,4-b]pyridinrings verbunden ist, und Ac den Acylrest einer Säure darstellt, sowie von pharmazeutisch verwendbaren Salzen von Verbindungen der Formel I mit salzbildenden Eigenschaften, als cardiotonisch wirksame und blutdrucksteigernde, sowie blutzuckersenkende Verbindungen.

Ein carbocyclischer oder heterocyclischer Arylrest R ist in erster Linie ein entsprechender monocyclischer Rest, kann jedoch auch ein bi- oder polycyclischer, carbocyclischer oder heterocyclischer Rest mit aromatischen Eigenschaften sein.

Carbocyclische Reste R dieser Art sind insbesondere gegebenenfalls substituiertes Phenyl, ferner Naphthyl.

Heterocyclische Arylreste R sind vorzugsweise entsprechende monocyclische Reste, können jedoch auch entsprechende bi- oder polycyclische Reste sein, wobei letztere aus mehreren heterocyclischen Ringen, oder aus einem oder mehreren heterocyclischen Ringen mit einem oder mehreren ankondensierten carbocyclischen Ringen, insbesondere einem oder mehreren ankondensierten Benzoringen bestehen können. Die üblicherweise vorliegenden heterocyclischen Reste R, die vorzugsweise aus fünf oder sechs Ringgliedern bestehen, können bis zu vier, gleiche oder verschiedene Hetero-, insbesondere Stickstoff-, Sauerstoff- und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem Kohlenstoffatom der 4-Stellung des Furo[3,4-b]pyridinrings üblicherweise über ein Ringkohlenstoffatom gebunden.

Monocyclische fünfgliedrige Heteroarylreste R sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-,

Isoxazolyl-, Oxazolyl-, Oxadiazolyl-, Isothiazolyl-, Thiazolyl- oder Thiadiazolylreste, während monocyclische, sechsgliedrige Heteroarylreste R z.B. entsprechende monoaza-, diaza- oder triazacyclische Reste, wie Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder sechsgliedrig sein, wobei ein fünfgliedriger Heteroarylrest z.B. einen monoaza-, diaza-, monooxa-, monothia-, oxaza- oder thiazacyclischen Rest, und ein sechsgliedriger Heteroarylrest z.B. einen monoaza- oder ein diazacyclischen Heteroarylrest darstellt. Solche bicyclischen Reste sind z.B. Indolyl-, Isoindolyl-, Benzimidazolyl-, Benzofuranyl-, Benzothienyl-, Benzthiazolyl-, Chinolinyl- oder Isochinolinylreste.

Die für die carbocyclischen und heterocyclischen Arylreste R genannten Substituenten stehen in erster Linie an Ringkohlenstoffatomen; es können aber auch Ringstickstoffatome substituiert sein. Substituenten von Ringstickstoffatomen sind u.a. gegebenenfalls substituierte Kohlenwasserstoffreste, wie entsprechende aliphatische, cycloaliphatische, aromatische oder araliphatische Kohlenwasserstoffreste, z.B. Niederalkyl, Niederalkenyl, Niederalkinyl, Niederalkylen, Cycloalkyl, Cycloalkylniederalkyl, Phenyl oder Phenylniederalkyl. Substituenten von solchen Kohlenwasserstoffresten, insbesondere von Niederalkyl, Phenyl oder Phenylniederalkyl, sind z.B. gegebenenfalls verätherte oder veresterte Hydroxygruppen, wie Hydroxy, gegebenenfalls, z.B. durch gegebenenfalls veräthertes doer verestertes Hydroxy, substituiertes Niederalkoxy, z.B. Niederalkoxy, Niederalkoxyniederalkoxy, oder Halogenniederalkoxy, gegebenenfalls, z.B. durch gegebenenfalls veräthertes oder verestertes Hydroxy, substituiertes Niederalkenyloxy, z.B. Niederalkenyloxy oder Halogenniederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes

Carboxy, z.B. Niederalkoxycarbonyl, amidiertes Carboxy, wie Carbamoyl, N-Niederalkyl-carbamoyl oder N,N-Diniederalkyl-carbamoyl, oder Cyan. Cyclische Substituenten, insbesondere Phenyl, können zudem auch Niederalkyl, das gegebenenfalls, z.B. wie angegeben, substituiert sein kann, als Substituenten enthalten. Weitere Substituenten von Ringstickstoffatomen sind Hydroxy oder Oxido.

Heterocyclische Arylreste R können, z.B. je nach Art der Substitution, in verschiederartigen tautomeren Formen vorliegen.

Ein substituierter Niederalkylrest $R_1$ enthält als Substituenten in erster Linie unsubstituiertes oder insbesondere mono- oder disubstituiertes Amino, wie Diniederalkylamino, N-Niederalkyl-N-phenylniederalkyl-amino oder gegebenenfalls durch Sauerstoff, Schwefel oder unsubstituierten oder, z.B. durch Niederalkyl, substituierten Stickstoff unterbrochenes Niederalkylenamino, wobei ein solcher Substituent vorzugsweise durch mindestens 2 Kohlenstoffatome vom Ringstickstoffatom getrennt ist.

Veräthertes Hydroxy als Substituent eines Niederalkylrestes $R_2$ ist in erster Linie Niederalkoxy, während verestertes Hydroxy insbesondere Halogen oder Niederalkanoyloxy bedeutet. Funktionell abgewandeltes Carboxy als entsprechender Substituent einer Niederalkylgruppe $R_2$ ist insbesondere verestertes Carboxy, wie Niederalkoxycarbonyl, amidiertes Carboxy, wie Carbamoyl, N-Niederalkyl-carbamoyl oder N,N-Diniederalkyl-carbamoyl, oder Cyan. Substituiertes Amino als Substituenten von Niederalkyl $R_2$ ist monosubstituiertes Amino, wie Niederalkylamino, oder vorzugsweise disubstituiertes Amino, wie Diniederalkylamino, N-Niederalkyl-N-phenylniederalkyl-amino oder gegebenenfalls, z.B. durch Niederalkyl oder 2-Oxo-1-imidazolidinyl, substituiertes und/oder durch Sauerstoff, Schwefel oder gegebenenfalls, z.B. durch Niederalkyl oder Acyl, substituierten Stickstoff unterbrochenes Niederalkylenamino.

Funktionell abgewandeltes Carboxy und substituiertes Amino als Gruppe $R_2$ haben z.B. die oben gegebenen Bedeutungen und sind in erster Linie Niederalkoxycarbonyl oder Cyan bzw. Niederalkylamino oder disubstituiertes Amino-niederalkylamino, wie Diniederalkylaminoniederalkylamino oder gegebenenfalls im Niederalkylenteil, z.B. durch Niederalkyl oder 2-Oxo-1-imidazolidinyl, substituiertes und/oder durch Sauerstoff, Schwefel oder gegebenenfalls, z.B. durch Niederalkyl oder Acyl, substituierten Stickstoff unterbrochenes Niederalkylenaminoniederalkylamino, ferner Diniederalkylamino, oder, gegebenenfalls im Niederalkylenteil, z.B. durch Niederalkyl, substituiertes und/oder durch Sauerstoff, Schwefel oder gegebenenfalls, z.B. durch Niederalkyl oder Acyl, substituierten Stickstoff unterbrochenes Niederalkylenamino.

Eine mit einem Niederalkylrest $R_1$ verbundene Aminogruppe $R_2$ kann mit diesem zusammen einen, über das Stickstoffatom mit dem Ringkohlenstoff verbundenen 1-Aza-niederalkylenrest, vorzugsweise mit 3 bis 5 Kettenatomen, bilden. Ein durch Niederalkyl $R_2$ mit der Acylgruppe Ac gebildetes 2-Oxa-1-oxo-niederalkylen enthält vorzugsweise drei oder vier Kettenatome.

Ein Acylrest Ac kann den entsprechenden Rest einer Carbonsäure, insbesondere Niederalkanoyl, ferner unsubstituiertes oder substituiertes, z.B. Niederalkyl, Niederalkoxy und/oder Halogen enthaltendes Benzoyl, oder einer organischen Sulfosäure, insbesondere Niederalkylsulfonyl oder unsubstituiertes oder substituiertes, z.B. Niederalkyl, Niederalkoxy und/oder Halogen, enthaltendes Phenylsulfonyl, ferner den entsprechenden Rest der Cyanwasserstoffsäure, d.h. Cyan darstellen. Acylreste Ac stehen jedoch in erster Linie für Acylreste von Monoestern, ferner von Monoamiden der Kohlensäure, wie unsubstituiertes oder substituiertes, z.B. freies oder veräthertes Hydroxy, wie Niederalkoxy, oder unsubstituiertes oder substituiertes Amino, wie z.B. oben beschriebenes Amino, und in erster Linie disubstituiertes Amino, wie Diniederalkylamino, N-Niederalkyl-N-phenylniederalkyl-amino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder, z.B. durch Niederalkyl, Niederalkoxy und/oder Halogen, substituiertes Phenyl enthaltendes Niederalkoxycarbonyl, ferner N-unsubstituiertes oder N-mono- oder N,N-disubstituiertes Carbamoyl, wie N-Niederalkylcarbamoyl, N,N-Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylen-carbamoyl.

Die vor-, sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen bis und mit 7, vorzugsweise bis und mit 4 Kohlenstoffatome enthalten.

Substituierte Reste können einen oder mehrere, insbesondere bis zu drei, gleiche oder verschiedene Substituenten enthalten; diese können irgendeine geeignete Stellung einnehmen.

Naphthyl kann 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl z.B. 1,2,3,4-1H-Tetrazol-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4-Oxazolyl, Oxadiazolyl z.B. 1,3,4-Oxadiazol-2-yl, Isothiazolyl z.B. 3-Isothiazolyl, Thiazolyl 2- oder 4-Thiazolyl, und Thiadiazolyl z.B. 1,3,4-Thiadiazol-2-yl.

Pyridyl ist 2-, 3- oder 4-Pyridyl, Pyridazinyl z.B. 3-Pyridazinyl, Pyrimidinyl 2-, 4- oder 5-Pyrimidinyl, Pyrazinyl, 2-Pyrazinyl und Triazinyl z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzothienyl z.B. 3-Benzothienyl, Benzthiazolyl z.B. 2-Benzthiazolyl, Chinolinyl z.B. 2- oder 4-Chinolinyl, und Isochinolinyl z.B. 1-Isochinolinyl.

Niederalkyl ist z.B. Methyl, Aethyl, n-Propyl, Isopropyl, n-Butyl, Isobutyl oder tert.-Butyl, ferner n-Pentyl, n-Hexyl oder n-Heptyl, während Niederalkenyl z.B. Allyl oder Methallyl, und Niederalkinyl z.B. Propargyl bedeutet.

Niederalkylen enthält z.B. drei bis fünf Kettenkohlenstoffatome und ist u.a. 1,3-Propylen oder 1,4-Butylen.

Cycloalkyl weist vorzugsweise 5 bis 7 Ringkohlenstoffatome auf und ist z.B. Cyclopentyl oder Cyclohexyl, wähtend Cycloalkylniederalkyl z.B. Cyclopropylmethyl, Cyclopentylmethyl oder Cyclohexylmethyl sein kann.

Phenylniederalkyl ist z.B. Benzyl oder 1- oder 2-Phenyläthyl.

Niederalkoxy steht in erster Linie für Methoxy, Aethoxy, n-Propyloxy, Isopropyloxy, n-Butyloxy, Isobutyloxy oder tert.-Butyloxy.

In einem Niederalkoxyniederalkoxyrest oder einem Niederalkylthioniederalkoxyrest ist die terminale Niederalkoxygruppe vorzugsweise durch mehr als ein Kohlenstoffatom vom Verknüpfungssauerstoffatom getrennt; solche Reste sind z.B. 2-Methoxyäthoxy oder 2-Aethoxy-äthoxy,

bzw. 2-Methylthioäthoxy oder 2-Aethylthioäthoxy.

In einem Halogen-niederalkoxyrest, wobei Trifluormethoxy ausgenommen ist, können ein oder mehrere Halogenatome, die vorzugsweise eine Atomnummer bis 35 aufweisen und besonders für Fluor und/oder Chlor stehen, vorhanden sein; solche Reste sind z.B. Difluormethoxy oder 1,1,2-Trifluor-2-chlor-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis 35 aufweisen und besonders für Fluor und/oder Chlor stehen, ist z.B. 1,2-Dichlor-vinyloxy.

Niederalkinyloxy ist z.B. Propargyloxy, während Niederalkylendioxy z.B. Methylendioxy oder Aethylendioxy bedeutet.

Niederalkanoyloxy ist z.B. Acetyloxy, Propionyloxy oder Pivaloyloxy.

Halogen hat vorzugsweise eine Atomnummer bis und mit 35 und ist insbesondere Fluor oder Chlor, ferner Brom, kann jedoch Iod sein.

Halogensubstituiertes Niederalkyl ist z.B. Trifluormethyl, 1,1,2-Trifluor-2-chlor-äthyl oder Chlormethyl.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert.-Butyloxycarbonyl.

N-Niederalkyl-carbamoyl ist z.B. N-Methyl-carbamoyl oder N-Aethylcarbamoyl, während N,N-Diniederalkyl-carbamoyl z.B. N,N-Dimethylcarbamoyl oder N,N-Diäthyl-carbamoyl ist.

N-Niederalkylamino ist z.B. N-Methylamino, N-Aethylamino, N-n-Propylamino oder N-Isopropylamino.

N,N-Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-methyl-N-(2-phenyläthyl)-amino darstellt.

Niederalkylenamino enthält vorzugsweise 4 bis 6 Ringkohlenstoffatome und ist z.B. Pyrrolidino oder Piperidino, während Oxaniederalkylenamino z.B. 4-Morpholino, Thianiederalkylenamino z.B. 4-Thiomorpholino, und gegebenenfalls azasubstituiertes Azaniederalkylenamino z.B. Piperazino, 4-Methylpiperazino, 4-Phenyl-piperazino, 4-Benzyl-piperazino oder 4-(2-Phenyläthyl)-piperazino darstellen kann.

Niederalkanoylamino ist z.B. Acetylamino oder Propionylamino.

Niederalkanoyl ist z.B. Formyl, Acetyl, Propionyl oder Pivaloyl.

Niederalkylthio ist z.B. Methylthio, Aethylthio, n-Propylthio oder Isopropylthio, wähtend Niederalkylsulfinyl z.B. Methylsulfinyl, und Niederalkylsulfonyl z.B. Methylsulfonyl oder Aethylsulfonyl bedeuten.

N-Niederalkylaminosulfonyl ist z.B. N-Methylaminosulfonyl, während N,N-Diniederalkylaminosulfonyl z.B. N,N-Dimethylaminosulfonyl ist.

In einem Halogen-niederalkylthiorest können ein oder mehrere Halogenatome, die vorzugsweise eine Atomnummer bis 35 aufweisen und besonders für Fluor und/oder Chlor stehen, vorhanden sein. Solche Reste sind z.B. Difluormethylmercapto oder 1,1,2-Trifluor-2-chlor-äthylmercapto.

In einer Aminoniederalkylgruppe $R_1$ ist Amino vorzugsweise durch mindestens 2 Kohlenstoffatome vom Verknüpfungskohlenstoffatom getrennt;

solche Reste sind in erster Linie 2-disubstituiertes Amino-niederalkyl, wie 2-Diniederalkylaminoäthyl, z.B. 2-Dimethylaminoäthyl oder 2-Diäthylaminoäthyl, 2-Niederalkylenaminoäthyl, z.B. 2-Pyrrolidino-äthyl oder 2-Piperidino-äthyl, 2-(4-Morpholino)-äthyl, oder 2-(4-Niederalkyl-piperazino)-äthyl, z.B. 2-(4-Methylpiperazino)-äthyl.

Durch 2-Oxo-1-imidazolidinyl substituiertes Niederalkylenamino ist z.B. 4-(2-Oxo-1-imidazolidinyl)-piperidino.

Acyl als Substituent des, einen Niederalkylenaminorest unterbrechenden Stickstoffatoms ist in erster Linie der Acylrest einer Carbonsäure, wie Niederalkanoyl, z.B. wie oben erwähnt, Benzoyl, Furoyl, z.B. 2-Furoyl, oder Thenoyl, z.B. 2-Thenoyl. Ein entsprechender Azaniederalkylenaminorest ist z.B. 4-Niederalkanoyl-piperazino, 4-Benzoylpiperazino, 4-(2-Furoyl)-piperazino oder 4-(2-Thenoyl)-piperazino.

Substituierte Niederalkylreste $R_2$ sind in erster Linie entsprechend substituierte Methylreste, z.B. Hydroxymethyl, Niederalkoxymethyl, Halogenmethyl, Niederalkoxycarbonylmethyl oder Cyanmethyl.

Diniederalkylaminoniederalkylamino ist z.B. 2-Dimethylaminoäthyl-amino, 2-Diäthylaminoäthyl-amino oder 3-Dimethylaminopropyl-amino, während entsprechend durch Sauerstoff, Schwefel oder gegebenenfalls substituierten Stickstoff unterbrochenes Niederalkylenamino-niederalkylamino z.B. 2-Pyrrolidinoäthyl-amino, 2-Piperidinoäthyl-amino, 2-(4-Morpholino)-äthyl-amino oder 2-(4-Methyl-piperazino)-äthyl-amino darstellt.

Ein 1-Azaniederalkylenrest, den eine Aminogruppe $R_2$ zusammen mit einem Niederalkylenrest $R_1$ bilden kann, ist in erster Linie 1-Aza-1,3-propylen, ferner 1-Aza-1,4-butylen, wobei in einem solchen Rest das Azastickstoffatom mit dem 2-Ringkohlenstoffatom und das Verknüpfungs-

kohlenstoffatom mit dem 1-Ringstickstoffatom des Furo[3,4-b]pyridinrings verbunden ist.

Ein durch einen Niederalkylrest $R_2$ zusammen mit dem benachbarten Acylrest Ac gebildetes 2-Oxa-1-oxo-niederalkylen ist in erster Linie 2-Oxa-1-oxo-1,3-propylen, wobei dessen 3-Kohlenstoffatom mit dem 2-Ringkohlenstoffatom und dessen Carbonylgruppe mit dem 3-Ringkohlenstoffatom des Furo[3,4-b]pyridinrings verbunden ist.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropyloxycarbonyl, Niederalkoxy-niederalkoxycarbonyl, z.B. 2-Methoxyäthoxycarbonyl, Diniederalkylamino-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenamino-niederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder (4-Niederalkyl-piperazino)-niederalkoxycarbonyl, z.B. 2-(4-Methylpiperazino)-äthoxycarbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste in welchen der Niederalkylenteil durch Sauerstoff, Schwefel oder unsubstituierten oder substituierten Stickstoff unterbrochen ist, z.B. 4-Morpholinocarbonyl, 4-Thiomorpholino-carbonyl, 1-Piperazinocarbonyl oder 4-Methyl-1-piperazino-carbonyl bedeutet.

Verbindungen der Formel I mit salzbildenden Eigenschaften, insbesondere solche mit basischen Gruppen, können in Form von Salzen, insbesondere von Säureadditionssalzen, in erster Linie entsprechenden pharmazeutisch verwendbaren Säureadditionssalzen vorliegen. Solche Salze sind z.B. diejenigen mit Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäure, z.B. Benzolsulfonsäure, 4-Methyl-benzolsulfonsäure oder Naphthalin-2-sulfonsäure, oder mit anderen sauren organischen Substanzen, wie Ascorbinsäure.

Je nach Art der Substitution bzw. Substituenten können die Verbindungen der Formel I in Form von Gemischen von Racematen, oder in Form von Racematen oder optisch aktiven Antipoden vorliegen.

Verbindungen vom Typ der Formel I, sowie Salze von solchen Verbindungen mit salzbildenden Eigenschaften sind bekannt; vgl. deutsche Offenlegungsschrift 26 29 892. Gemäss dieser sollen sie, ebenso wie die zusammen mit ihnen beschriebenen 1,4-Dihydro-pyridin-3,5-dicarbonsäureester-Verbindungen, vasodilatatorische und antihypertensive Eigenschaften aufweisen und entsprechend zur Behandlung, z.B. des Bluthochdrucks, verwendet werden können.

Es ist nun gefunden worden, dass die Verbindungen der Formel I und Salze von solchen Verbindungen mit salzbildenden Eigenschaften, im Gegensatz zu den entsprechenden 1,4-Dihydro-pyridin-3,5-dicarbonsäure-diester-Verbindungen, eine Steigerung der myocardialen Kontraktilität (positiv inotrope Wirkung) und des Blutdrucks, sowie eine Senkung der Glucosekonzentration im Blut bewirken, und deshalb entsprechend als Blutdruck-steigernde und cardiotonisch-wirksame, ferner als blutzucker-senkende Mittel verwendet werden können.

Die Steigerung der myocardialen Kontraktilität, d.h. die positiv inotrope Wirkung, kann z.B. anhand von Versuchsanordnungen nachgewiesen werden, die sich auf die von J.V. Levy, Isolated Atrial Preparations in Methods in Pharmacology, Bd. 1, S. 77 bis 104 (Ed. A. Schwartz; Appleton-Century-Crofts, New York; 1971), beschriebene Methodik stützen. So steigern die Verbindungen der Formel I und Salze von solchen Verbindungen mit salzbildenden Eigenschaften in Konzentrationen ab etwa 0,1 µmol/L an isolierten, elektrisch gereizten, linken Vorhöfen des Meerschweinchens (in vitro-Versuchsanordnung) die maximale Kontraktionskraft (peak developed tension). Das Ausmass (efficacy) dieser positiv inotropen Wirkung ist dabei unterschiedlich, kann jedoch bis zu 90 % der mit Isoproterenol maximal erreichbaren Wirkung betragen. Ferner steigern sie in Konzentrationen ab etwa 0,3 µ mol/L an isolierten, spontan schlagenden, rechten Vorhöfen des Meerschweinchens die Kontraktionsfrequenz. Das Ausmass (efficacy) dieser positiv chronotropen Wirkung ist dabei unterschiedlich, kann jedoch bis zu 60% der mit Isoproterenol maximal erreichbaren Wirkung betragen.

Im Gegensatz dazu zeigen entsprechende 1,4-Dihydro-pyridin-3,5-dicarbonsäure-diester-Verbindungen, wie z.B. der 2-Cyan-6-methyl-4-(3-nitrophenyl)-1,4-dihydro-pyridin-3,5-dicarbonsäure-5-isopropylester-3-methylester, der z.B. unter den Schutzumfang der obgenannten deutschen Offenlegungsschrift 2 629 892 fällt und spezifisch in der deutschen

Offenlegungsschrift 2 940 833 beschrieben wird, sowie das als Vergleichssubstanz herangezogene Nifedipin (2,6-Dimethyl-4-(2-nitrophenyl)-1,4-dihydro-pyridin-3,5-dicarbonsäure-dimethylester) in der gleichen Versuchsanordnung ausschliesslich eine Herabsetzung der Kontraktionskraft, d.h. eine negativ inotrope Wirkung bzw. eine Herabsetzung der Kontraktionsfrequenz, d.h. eine negativ chronotrope Wirkung.

In der folgenden Tabelle werden die Konzentrationen (in µmol/L) für Verbindungen der Formel I angegeben, die notwendig sind, um die Hälfte der maximalen positiv inotropen Wirkung zu erzielen ($EC_{50}$). Das Ausmass der Wirkung wird in % der maximalen, mittels Isoproterenol in dieser Versuchsanordnung bewirkten Steigerung der Kontraktionskraft ($ISO_{max.}$) angegeben; diejenige von Isoproterenol beträgt +2.1 ±0.1 g ($\bar{x} \pm S_{\bar{x}}$; N=30).

| Verbindungen der Formel I ($R_1$ = Wasserstoff; $R_2$ = Methyl) | | Wirksamkeit $EC_{50}$ (µmol/L) | Ausmass der Wirkung (% von $ISO_{max}$) |
|---|---|---|---|
| Ac | R | | |
| $-COOC_2H_5$ | 2-Difluormethoxyphenyl | 0.7 | 65 |
| $-COOC_2H_5$ | 2-(2-Chlor-1,1,2-trifluor-äthoxy)-phenyl | 1.0 | 75 |
| $-COOC_2H_5$ | 4-Difluormethoxyphenyl | | 10 |
| $-COOC_2H_5$ | 2,3-Di-difluormethoxyphenyl | | 14 |
| -CN | 2-Difluormethoxyphenyl | 63 | 68 |
| $-COOC_2H_5$ | 2-Difluormethylmercaptophenyl | 1.0 | 80 |
| $-COOC_2H_5$ | 3-Difluormethoxyphenyl | 4.8 | 65 |

| Verbindungen der Formel I ($R_1$ = Wasserstoff; $R_2$ = Methyl) Ac | R | Wirksamkeit $EC_{50}$ (µmol/L) | Ausmass der Wirkung (% von $ISO_{max}$) |
|---|---|---|---|
| $-COOCH_3$ | 2-Difluormethoxy-phenyl | 3.7 | 70 |
| $-COOC_2H_4OCH_3$ | 2-Difluormethoxy-phenyl | 5.3 | 90 |
| $-COOCH(CH_3)_2$ | 2-Difluormethoxy-phenyl | 1.2 | 70 |
| $-COOC_2H_5$ | 2-(2-Methoxyäthoxy)-phenyl | 3.8 | 80 |
| $-COOC_2H_5$ | 2-(2-Methylmercapto-äthoxy)-phenyl | 0.38 | 75 |

Im Gegensatz dazu weist z.B. der obgenannte 2-Cyan-6-methyl-4-(3-nitrophenyl)-1,4-dihydro-pyridin-3,5-dicarbonsäure-5-isopropylester-3-methylester in der gleichen Versuchsanordnung eine Reduktion der Kontraktionskraft um 25 % bei einer Konzentration von 1 µmol/L und um 50 % bei einer solchen von 8 µmol/L auf, während Nifedipin bei einer Konzentration von 0.1 µmol/L eine 25 %ige und von 0.4 µmol/l eine 50 %ige Reduktion der myocardialen Kontraktionskraft bewirkt.

Im narkotisierten Hund (in vivo-Versuchsanordnung) bewirken die Verbindungen der Formel I und Salze von solchen Verbindungen mit salzbildenen Eigenschaften bei intravenöser Verabreichung in Dosen ab etwa 1 mg/kg ebenfalls eine Steigerung der myokardialen Kontraktilität (Zunahme der maximalen Druckanstiegsgeschwindigkeit $dP/dt_{max}$ in der Aorta) um etwa 40 bis etwa 60 %, sowie eine Steigerung des arteriellen Blutdrucks um etwa 10 bis etwa 30 %, ferner eine Abnahme der Glucose-Konzentration im Blut um etwa 10 bis etwa 30 %. Im Gegensatz dazu bewirkt der erwähnte 1,4-Dihydro-pyridin-3,5-dicarbonsäurediester-Verbindungen, wie der 2,6-Dimethyl-4-(2-nitrophenyl)-1,4-dihydro-pyridin-3,5-dicarbonsäuredimethylester im narkotisierten Hund eine Senkung des Blutdrucks.

An der narkotisierten Katze führen die Verbindungen der Formel I und Salze von solchen Verbindungen mit salzbildenden Eigenschaften bei intravenöser Verabreichung in Dosen ab etwa 0,001 mg/kg zu einer Steigerung des arteriellen Blutdrucks. Im Gegensatz dazu verursachen z.B. die obgenannten 2,6-Dimethyl-4-(2-nitro-phenyl)-1,4-dihydro-pyridin-3,5-dicarbonsäuredimethylester und 2-Cyan-6-methyl-4-(3-nitro-phenyl)-1,4-dihydro-pyridin-3,5-dicarbonsäure-5-isopropylester-3-methylester in der gleichen Versuchsanordnung bei intravenöser Verabreichung in Dosen ab etwa 0,001 mg/kg eine Senkung des arteriellen Blutdrucks.

Die Verbindungen der Formel I und Salze von solchen Verbindungen mit salzbildenden Eigenschaften können deshalb als Blutdruck-steigernde Mittel zur Behandlung von Zuständen mit arterieller Hypotonie (z.B. vegetative Dystonie), sowie als cardiotonische Mittel zur Behandlung von Zuständen mit verminderter Pumpleistung des Herzens, z.B. cardiogener Schock oder Schockzustände anderer Genese, ferner als blutzuckersenkende Mittel, z.B. zur Behandlung von Diabetes mellitus, verwendet werden.

Die Verbindungen der Formel I sowie Salze von solchen Verbindungen mit salzbildenden Eigenschaften wirken ferner aktivierend, insbesondere stimulierend, auf das Zentralnervensystem. So bewirkt z.B. die i.p.-Verabreichung von 50 mg/kg 4-(2-Difluormethoxyphenyl)-2-methyl-5-oxo-1,4,5,7-tetrahydro-furo[3,4-b]pyridin-3-carbonsäureäthylester als Lösung in Polyäthylenglycol 400 an männliche Ratten während 1 Stunde, insbesondere während 30 Minuten nach Verabreichung Schnuppern, Kau- und Putzaktivität, sporadische Schüttelbewegungen von Kopf und Körper sowie Vorderpfoten-Klonus. Aufgrund dieser Eigenschaften erscheinen die Verbindungen der Formel I sowie Salze von solchen Verbindungen mit salzbildenden Eigenschaften für die Behandlung retardierter Depressionen verschiedenen Schweregrades, von psychischen, von fehlendem Antrieb und Initiative begleiteten Störungen sowie von hypokinetischen, geriatrischen Patienten geeignet.

Die Erfindung betrifft insbesondere neue Verbindungen der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pridazinyl, Pyrimidinyl, Pyrazinyl, Triazinyl, Indolyl, Isoindolyl, Benzimidazolyl, Benzofuranyl, Benzothienyl, Chinolinyl oder Isochinolinyl bedeutet, wobei in diesen Resten Ringkohlenstoffatome durch Niederalkoxyniederalkoxy, Niederalkylthioniederalkoxy, Halogenniederalkoxy, ausgenommen Trifluormethoxy, oder Halogenniederalkylthio substituiert sind, und Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy,

Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, $R_1$ Wasserstoff, Niederalkyl, Diniederalkylamino-niederalkyl, Niederalkylenaminoniederalkyl, Morpholinoniederalkyl, Thiomorpholinoniederalkyl, Piperazinoniederalkyl oder 4-Niederalkyl-piperazinoniederalkyl darstellt, $R_2$ für Wasserstoff, Niederalkyl, Hydroxyniederalkyl, Niederalkoxyniederalkyl, Halogenniederalkyl, Niederalkoxycarbonylniederalkyl, Carbamoylniederalkyl, N-Niederalkylcarbamoylniederalkyl, N,N-Diniederalkylcarbamoylniederalkyl, Cyanniederalkyl, Aminoniederalkyl, Niederalkylaminoniederalkyl, Diniederalkylaminoniederalkyl, Niederalkylenaminoniederalkyl, Morpholinoniederalkyl,

Thiomorpholinoniederalkyl, Piperazinoniederalkyl, 4-Niederalkyl-piperazino-niederalkyl, Niederalkoxycarbonyl, Carbamoyl, N-Niederalkylcarbamoyl, N,N-Diniederalkylcarbamoyl, Cyan, Amino, Niederalkylamino, Diniederalkylamino-niederalkylamino, Niederalkylenamino-niederalkylamino, Morpholino-niederalkylamino, Diniederalkylamino, Niederalkylenamino, (2-Oxo-1-imidazolidinyl)-niederalkylenamino, Morpholino, Thiomorpholino, Piperazino, 4-Niederalkyl-piperazino, 4-Niederalkanoyl-piperazino, 4-Benzoyl-piperazino, 4-Furoylpiperazino oder 4-Thenoyl-piperazino steht, wobei eine Aminogruppe $R_2$ mit einem Niederalkylrest $R_1$ verbunden sein und mit diesem zusammen einen 1-Aza-niederalkylenrest bilden kann, dessen Stickstoffatom mit dem 2-Ringkohlenstoffatom des Furo[3,4-b]pyridinrings verbunden ist, oder, falls $R_2$ für Niederalkyl steht, dieses zusammen mit dem benachbarten Acylrest Ac einen 2-Oxa-1-oxo-niederalkylenrest bilden kann, dessen Carbonylgruppe mit dem 3-Ringkohlenstoffatom des Furo[3,4-b]-pyridinrings verbunden ist, und der Rest Ac für Niederalkanoyl, unsubstituiertes oder durch Niederalkyl, Niederalkoxy und/oder Halogen substituiertes Benzoyl, Niederalkylsulfonyl, unsubstituiertes oder durch Niederalkyl, Niederalkoxy und/oder Halogen substituiertes Phenylsulfonyl, Cyan, Niederalkoxycarbonyl, Hydroxyniederalkoxycarbonyl, Niederalkoxyniederalkoxycarbonyl, Aminoniederalkoxycarbonyl, Niederalkylaminoniederalkoxycarbonyl, Diniederalkylaminoniederalkoxycarbonyl, N-Niederalkyl-N-phenylniederalkyl-amino-niederalkoxycarbonyl, Niederalkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy und/oder Halogen substituiertes Phenylniederalkoxycarbonyl, Carbamoyl, N-Niederalkyl-carbamoyl, N,N-Diniederalkylcarbamoyl, N,N-Niederalkylencarbamoyl, Morpholinocarbonyl, Thiomorpholinocarbonyl, Piperazinocarbonyl oder 4-Niederalkyl-piperazinocarbonyl steht, oder pharmazeutisch verwendbare Salze von solchen Verbindungen mit salzbildenden Eigenschaften, in erster Linie pharmazeutisch verwendbare Säureadditionssalze von solchen Verbindungen mit salzbildenden basischen Eigenschaften.

Die Erfindung betrifft in erster Linie neue Verbindungen der Formel I, worin R für Phenyl, Naphthyl, Pyrryl, Furyl, Thienyl, Pyridyl oder über ein Ringkohlenstoff gebundenes Imidazolyl steht, die Niederalkoxyniederalkoxy, Niederalkylthioniederalkoxy, Halogenniederalkoxy, ausgenommen Trifluormethoxy, oder Halogenniederalkylthio als Substituenten enthalten, $R_1$ Wasserstoff, Niederalkyl, Diniederalkylamino-niederalkyl, Niederalkylenaminoniederalkyl oder (4-Morpholino)-niederalkyl darstellt, wobei Diniederalkylamino, Niederalkylenamino bzw. 4-Morpholino durch mindestens zwei Kohlenstoffatome vom Ringstickstoffatom getrennt sind, $R_2$ für Wasserstoff, Niederalkyl, Hydroxyniederalkyl, Halogenniederalkyl, Niederalkoxycarbonylniederalkyl, Cyanniederalkyl, Diniederalkylaminoniederalkyl, Niederalkoxycarbonyl, Cyan, Amino, (4-Morpholino)-niederalkylamino, Niederalkylenamino, (2-Oxo-1-imidazolidinyl)-niederalkylenamino oder 4-(2-Furoyl)-piperazino bedeutet, wobei eine Aminogruppe $R_2$ mit einem Niederalkylrest $R_1$ verbunden sein und mit diesem zusammen einen 1-Aza-niederalkylenrest bilden kann, dessen Azastickstoffatom mit dem 2-Ringkohlenstoffatom des Furo[3,4-b]pyridinrings verbunden ist, oder, falls $R_2$ für Niederalkyl steht, dieses zusammen mit dem benachbarten Acylrest Ac einen 2-Oxa-1-oxo-niederalkylenrest bilden kann, dessen Carbonylgruppe mit dem 3-Ringkohlenstoffatom des Furo[3,4-b]pyridinrings verbunden ist, und der Rest Ac für Niederalkanoyl, Niederalkylsulfonyl, Cyan, Niederalkoxycarbonyl, Hydroxyniederalkoxycarbonyl, Niederalkoxyniederalkoxycarbonyl, N,N-Diniederalkylaminoniederalkoxycarbonyl, N-Niederalkyl-N-phenylniederalkyl-amino-niederalkoxycarbonyl, N,N-Niederalkylenaminoniederalkoxycarbonyl, (4-Morpholino)-niederalkoxycarbonyl, Carbamoyl, N-Niederalkyl-carbamoyl, N,N-Diniederalkyl-carbamoyl, N,N-Niederalkylencarbamoyl, 4-Niederalkyl-piperazino-carbonyl oder 4-Morpholinocarbonyl steht, oder pharmazeutisch verwendbare Salze von solchen Verbindungen mit salzbildenden Eigenschaften, in erster Linie pharmazeutisch verwendbare Säureadditionssalze von solchen Verbindungen mit salzbildenden basischen Eigenschaften.

Die Erfindung betrifft insbesondere neue Verbindungen der Formel I, worin R für Phenyl, Pyridyl, z.B. 2-, 3- oder 4-Pyridyl, Furyl, z.B. 2-Furyl, oder Thienyl, z.B. 2-Thienyl steht, die durch Niederalkoxyniederalkoxy, z.B. Methoxymethoxy, oder 2-Methoxyäthoxy, Niederalkylthioniederalkoxy, z.B. 2-Methylthioäthoxy, Halogenniederalkoxy, ausgenommen Trifluormethoxy, z.B. Difluormethoxy oder 2-Chlor-1,1,2-trifluoräthoxy, oder Halogenniederalkylthio, z.B. Difluormethylmercapto, substituiert sind, wobei ein Phenylrest einen oder mehrere, gleiche oder verschiedene Substituenten aufweist, $R_1$ insbesondere Wasserstoff, ferner Niederalkyl, z.B. Methyl, 2-(Diniederalkylamino)-niederalkyl, besonders 2-Dimethylaminoäthyl, 2-(Niederalkylenamino)-niederalkyl, besonders 2-(Pyrrolidino)-äthyl oder 2-(Piperidino)-äthyl, oder 2-(4-Morpholino)-niederalkyl, besonders 2-(4-Morpholino)-äthyl, bedeutet, $R_2$ Niederalkyl, besonders Methyl, Hydroxyniederalkyl, besonders Hydroxymethyl, Halogenniederalkyl, insbesondere Chlormethyl, 2-(Diniederalkylamino)-niederalkyl, insbesondere 2-(Diniederalkylamino)-äthyl, Niederalkoxycarbonyl, besonders Methoxycarbonyl oder Aethoxycarbonyl, Cyan, Amino, (4-Morpholino)-niederalkylamino, besonders 2-(4-Morpholino)-äthylamino, Niederalkylenamino, besonders Pyrrolidino, (2-Oxo-1-imidazolidinyl)-niederalkylenamino, besonders 4-(2-Oxo-1-imidazolidinyl)-piperidino, oder 4-(2-Furoyl)-piperazino bedeutet, wobei eine Aminogruppe $R_2$ mit einem Niederalkylrest $R_1$ verbunden und zusammen mit diesem einen 1-Azaniederalkylenrest, insbesondere 1-Aza-1,3-propylen bilden kann, dessen Azastickstoffatom mit dem 2-Ringkohlenstoffatom des Furo-[3,4-b]pyridinrings verbunden ist, oder, falls $R_2$ für Niederalkyl, besonders Methyl steht, dieses zusammen mit dem benachbarten Acylrest Ac einen 2-Oxa-1-oxo-niederalkylenrest, besonders 2-Oxa-1-oxo-1,3-propylen bilden kann, dessen Carbonylgruppe mit dem 3-Ringkohlenstoffatom des Furo[3,4-b]pyridinrings verbunden ist, und der Rest Ac Niederalkanoyl, z.B. Acetyl, Niederalkylsulfonyl, z.B. Methylsulfonyl oder Aethylsulfonyl, Niederalkoxycarbonyl, besonders Methoxycarbonyl, Aethoxycarbonyl, n-Propyloxycarbonyl, Isopropyloxycarbonyl oder Isobutyloxycarbonyl, 2-Niederalkoxyniederalkoxycarbonyl, insbesondere 2-Methoxyäthoxycarbonyl, 2- oder 3-Diniederalkylamino-niederalkoxycarbonyl, wie

2-Dimethylaminoäthoxycarbonyl, 2-Diäthylaminoäthoxycarbonyl oder 3-Dimethylaminopropyloxycarbonyl, 2- oder 3-(N-Niederalkyl-N-phenylniederalkyl-amino)-niederalkoxycarbonyl, z.B. 2-(N-Benzyl-N-methylamino)-äthoxycarbonyl, Carbamoyl, N-Niederalkyl-carbamoyl, z.B. N-Methylcarbamoyl, N,N-Diniederalkyl-carbamoyl, z.B. N,N-Dimethylcarbamoyl, N,N-Niederalkylenamino-carbonyl, z.B. Pyrrolidino-carbonyl, 4-Niederalkyl-piperazino-carbonyl, z.B. 4-Methyl-piperazino-carbonyl, oder 4-Morpholino-carbonyl oder Cyan bedeutet, oder pharmazeutisch verwendbare Salze von solchen Verbindungen mit salzbildenden Eigenschaften, in erster Linie pharmazeutisch verwendbare Säureadditionssalze von solchen Verbindungen mit salzbildenden basischen Eigenschaften.

Die Erfindung betrifft insbesondere neue Verbindungen der Formel I, worin R für durch Niederalkoxyniederalkoxy, z.B. 2-Methoxyäthoxy, Niederalkylthioniederalkoxy, z.B. 2-Methylthioäthoxy, Halogenniederalkoxy, ausgenommen Trifluormethoxy, worin Halogen eine Atomnummer bis und mit 35 hat und insbesondere Fluor oder Chlor ist, z.B. Difluormethoxy oder 2-Chlor-1,1,2-difluor-äthoxy, oder durch Halogenniederalkylthio, z.B. Difluormethylmercapto, substituiertes Phenyl steht, $R_1$ Wasserstoff bedeutet, $R_2$ Niederalkyl, z.B. Methyl bedeutet, und der Rest Ac in erster Linie Niederalkoxycarbonyl, z.B. Methoxycarbonyl, Aethoxycarbonyl, n-Propyloxycarbonyl, Isopropyloxycarbonyl oder Isobutyloxycarbonyl, ferner 2-Niederalkoxyniederalkoxycarbonyl, z.B. 2-Methoxyäthoxycarbonyl, darstellt.

Die Erfindung betrifft insbesondere neue Verbindungen der Formel I, worin R für in 3-, insbesondere aber in 2-Stellung durch Difluormethoxy, oder 2-Chlor-1,1,2-trifluoräthoxy, oder für in 3-Stellung, insbesondere aber in 2-Stellung durch Halogenniederalkylthio, z.B. Difluormethylmercapto, oder für in 3-Stellung, insbesondere aber in 2-Stellung durch Niederalkoxyniederalkoxy, z.B. 2-Methoxyäthoxy, oder Niederalkylthioniederalkoxy, z.B. 2-Methylthioäthoxy, substituiertes Phenyl steht $R_1$ Wasserstoff bedeutet, $R_2$ Niederalkyl, z.B. Methyl ist, und der Rest Ac Niederalkoxycarbonyl z.B. Methoxycarbonyl, Aethoxycarbonyl oder Isopropyloxycarbonyl, oder Niederalkoxyniederalkoxycarbonyl, z.B. 2-(Methoxy)-äthoxycarbonyl, darstellt.

Die Erfindung betrifft insbesondere die neuen, in den Beispielen beschriebenen spezifischen Verbindungen.

Die neuen Verbindungen der Formel I und Salze von solchen Verbindungen mit salzbildenden Eigenschaften können in an sich bekannter Weise hergestellt werden, indem man z.B.

a) eine Verbindung der Formel

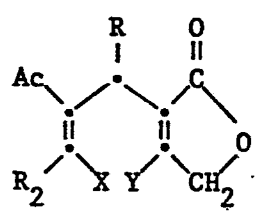

(II)

worin einer der Reste X und Y für die Gruppe der Formel $-NH-R_1$ steht und der andere Hydroxy oder die Gruppe der Formel $-NH-R_1$ bedeutet, oder ein Tautomeres davon oder ein entsprechendes Tautomerengemisch ringschliesst, oder

b) eine Verbindung der Formel

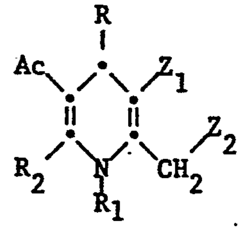

(III)

0111455



worin $Z_1$ eine freie, veresterte oder anhydridisierte Carboxylgruppe bedeutet, und $Z_2$ für eine gegebenenfalls reaktionsfähige veresterte Hydroxygruppe steht, ringschliesst, oder

c) eine Verbindung der Formel R-CHO (IV) oder ein reaktionsfähiges funktionelles Derivat davon mit einer Verbindung der Formel

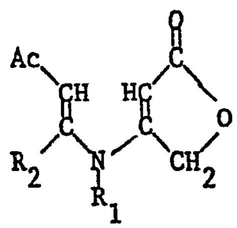

(V)

oder einem Tautomeren davon oder einem entsprechenden Tautomerengemisch umsetzt, oder

d) in einer Verbindung der Formel

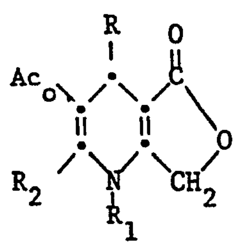

(VI)

worin $Ac_o$ einen in die Gruppe Ac überführbaren Rest darstellt, diesen in letztere überführt, wobei in den obigen Ausgangsstoffen der Formeln II bis VI, die, sofern sie salzbildende Eigenschaften aufweisen, auch in Form ihrer Salze verwendet werden können, die Reste R, $R_1$, $R_2$ und Ac die unter der Formel I gegebenen Bedeutungen haben, und wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die

einzelnen Isomeren oder ein erhaltenes Racemat in die optischen Antipoden auftrennt.

Ueblicherweise werden die in der Verfahrensvariante a) verwendeten Ausgangsstoffe der Formel II in situ gebildet, und der verfahrensgemässe Ringschluss findet unter den Reaktionsbedingungen für die Herstellung des Augsgangsmaterials statt. So kann man die Ausgangsstoffe der Formel II und unter den Reaktionsbedingungen üblicherweise auch die entsprechenden Endprodukte der Formel I erhalten, indem man aa) eine Verbindung der Formel IV oder ein reaktionsfähiges funktionelles Derivat davon mit einer Verbindung der Formel

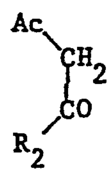

(VII),

einer Verbindung der Formel

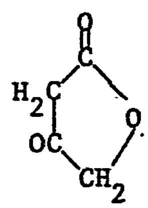

oder

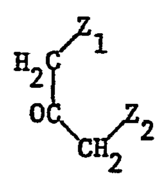

(VIII) (IX)

und einer Verbindung der Formel $R_1$-$NH_2$ (X) umsetzt, oder ab) eine Verbindung der Formel IV oder ein reaktionsfähiges funktionelles Derivat davon mit einer Verbindung der Formel

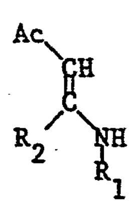

(XI),

und einer Verbindung der Formel VIII oder IX umsetzt, oder ac) eine Verbindung der Formel IV oder ein reaktionsfähiges funktionelles Derivat davon mit einer Verbindung der Formel

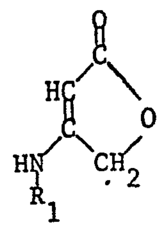 oder 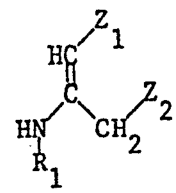

(XII) (XIII)

und einer Verbindung der Formel VII oder XI umsetzt, oder ad) eine Verbindung der Formel X mit einer Verbindung der Formel

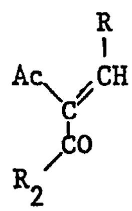

(XIV)

und einer Verbindung der Formel VIII oder IX umsetzt, oder ae) eine Verbindung der Formel X mit einer Verbindung der Formel

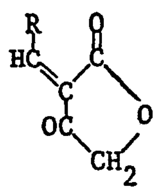 oder 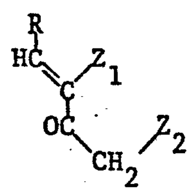

(XV) (XVI)

und einer Verbindung der Formel VII oder XI umsetzt, oder af) eine Verbindung der Formel X mit einer Verbindung der Formel

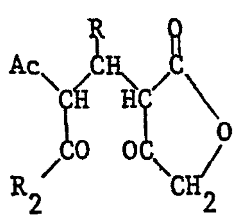 oder 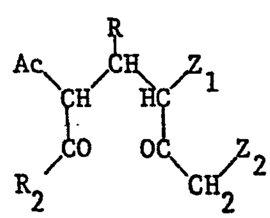

(XVII) (XVIII),

umsetzt, oder ag) eine Verbindung der Formel XI mit einer Verbindung der Formel XV oder XVI oder der Formel

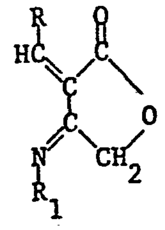

oder

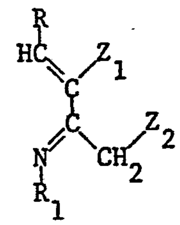

(XIX) (XX)

umsetzt, oder ah) eine Verbindung der Formel XII oder XIII mit einer Verbindung der Formel XIV oder mit einer Verbindung der Formel

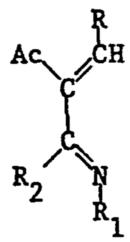

(XXI)

umsetzt. Dabei können mit Ausnahme der Verbindungen der Formeln IV und X die Verbindungen der Formeln VII bis IX und XI bis XXI in Form von Tautomeren oder in Form von Tautomerengemischen verwendet werden; Ausgangsstoffe der obigen Formeln mit salzbildenden Eigenschaften können auch in Form von Salzen verwendet werden. Ferner haben in den obgenannten Verbindungen die Gruppen R, $R_1$, $R_2$ und Ac die im Zusammenhang mit den Definitionen betr. Formel I und $Z_1$ und $Z_2$ die im Zusammenhang mit den Definitionen betr. Formel III gegebenen Bedeutungen.

Reaktionsfähige funktionelle Derivate des Aldehyds der Formel IV sind u.a. die entsprechenden Acetale, wie Diniederalkyl-, z.B. Dimethyl- oder Diäthylacetale, Acylale wie Diniederalkanoylacyclale, z.B. Diacetylacylale, oder die entsprechenden Dihalogenmethyl-, z.B. Dichlormethyl- oder Dibrommethyl-Verbindungen, ferner Additionsverbindungen, wie solche mit Wasser oder einem Alkalimetall-, z.B. Kaliumhydrogensulfit.

Die Gruppe $Z_1$ ist in erster Linie eine veresterte Carboxylgruppe, insbesondere Niederalkoxycarbonyl, z.B. Methoxycarbonyl oder Aethoxycarbonyl, kann aber auch substituiertes, z.B. durch Phenyl

oder funktionelle Gruppen, wie gegebenenfalls veräthertes oder verestertes Hydroxy substituiertes Niederalkoxycarbonyl sein. Eine anhydridisierte Carboxygruppe ist vorzugsweise eine, mit einer Mineralsäure eine gemischte anhydridisierte Carboxygruppe, z.B. eine Halogen-, insbesondere Chlorcarbonylgruppe bildende Carboxygruppe. Eine reaktionsfähige verestere Hydroxygruppe $Z_2$ ist insbesondere eine mit einer Mineralsäure, z.B. einer Halogenwasserstoffsäure, verestere Hydroxygruppe und steht insbesondere für Halogen, z.B. Chlor, Brom oder Iod; sie kann jedoch auch durch eine geeignete organische Carbon- oder Sulfonsäure verestert sein, und z.B. Methansulfonyloxy oder p-Toluolsulfonyloxy darstellen.

Eine Verbindung der Formel X kann auch in Form eines, diese Verbindung in situ abgegebenen Mittels, z.B. Ammoniak in Form eines Ammoniumsalzes, wie Ammoniumacetat oder Ammoniumhydrogencarbonat, oder einer Leichtmetall-, z.B. Alkalimetallverbindung, wie Natriumamid oder Lithium-N-methylamid, verwendet werden.

Bei der Ringschlussreaktion a), sowie den Kondensationsreaktionen aa) bis ah) zur Herstellung des üblicherweise in situ gebildeten Ausgangsmaterials für die Ringschlussreaktion, handelt es sich um Varianten der Dihydropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der Umsetzung von Verbindungen der Formel IV mit Verbindungen der Formeln XII bzw. XIII und XI gemäss Variante ac), von Verbindungen der Formel XI mit Verbindungen der Formel XIX bzw. XX gemäss Variante ag), oder von Verbindungen der Formel XII bzw. XIII mit Verbindungen der Formel XXI gemäss Variante ah) wird zusätzlich zu bzw. anstelle von Wasser eine Verbindung der Formel X abgespalten.

Falls nach der Variante aa) Verbindungen der Formel I hergestellt werden sollen, können unerwünschte Nebenprodukte, z.B. vom Typ der 1,4-Dihydro-pyridin-3,5-dicarbonsäurederivate, wie entsprechende Diester, entstehen. Durch nicht-gleichzeitiges Zusammengeben der Reaktionsteilnehmer kann die Bildung solcher Nebenprodukte indessen herabgesetzt werden, indem man einen bestimmten Reaktionsverlauf fördert, der in situ gemäss einer anderen Variante verläuft, da entsprechend der gestaffelten Zugabe der Reaktionskomponenten z.B. zunächst eine Verbindung der allgemeinen Formel XI oder der Formel XII bzw. XIII entstehen kann.

Die verfahrensgemässen Ringschluss- bzw. Kondensationsreaktionen werden in an sich bekannter Weise durchgeführt, falls notwendig, in Gegenwart eines Kondensationsmittels, insbesondere eines basischen Kondensationsmittels, wie eines Ueberschusses einer basischen Reaktionskomponente oder einer zusätzlichen, z.B. organischen Base, wie Piperidin oder Aethyl-diisopropyl-amin, oder eines Metallalkoholats, wie Alkalimetall-niederalkanolats, oder, falls eine Verbindung der Formel X als eine solche mit einem Leichtmetall, etwa als Natriumamid, vorliegt, in Gegenwart saurer Mittel, etwa einer organischen Carbonsäure, z.B. Essigsäure, und/oder eines geeigneten Dehydratisierungs- oder Wasser-aufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder eines Ausgangsstoffes der Formel X, im geschlossenen Gefäss unter erhöhtem Druck.

Unter den Reaktionsbedingungen können Verbindungen der Formeln IX, XIII, XVI, XVIII und XX in die entsprechenden Verbindungen der

Formeln VIII, XII, XV, XVII bzw. XIX umgewandelt, und die im Verlauf der Reaktion in situ gebildeten Zwischenprodukte, die den Lactonring noch nicht aufweisen, wie z.B. ein Zwischenprodukt der Formel

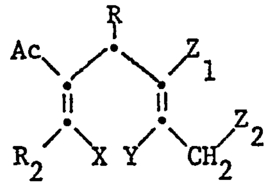

(XXII)

durch Ringschluss in Zwischenprodukte mit dem Lactonring, u.a. in ein Ausgangsmaterial der Formel II, übergeführt werden.

Die in den Verfahrensvarianten verwendeten Ausgangsstoffe sind bekannt oder können nach an sich bekannten Verfahren hergestellt werden.

Im Ausgangsmaterial der Formel III der Verfahrensvariante (b) haben die Gruppen $Z_1$ und $Z_2$ die oben angegebenen Bedeutungen, wobei $Z_1$ in erster Linie für Niederalkoxycarbonyl und $Z_2$ für Hydroxy oder Halogen, z.B. Chlor, steht.

Die Ringschlussreaktion wird in an sich bekannter Weise durchgeführt, in Ab- oder üblicherweise in Anwesenheit eines geeigneten Lösungs- oder Verdünnungsmittels oder eines entsprechenden Gemisches und/oder eines Kondensationsmittels, wobei man unter Kühlen bei Raumtemperatur, oder vorzugsweise unter Erwärmen, z.B. in einem Temperaturbereich von etwa 0°C bis etwa 200°C, vorzugsweise von etwa 40°C bis etwa 150°C, und, falls notwendig, in einem geschlossenen Gefäss, gegebenenfalls unter Druck und/oder unter einer Inertgasatmosphäre arbeitet.

Das Ausgangsmaterial der Formel III kann in an sich bekannter Weise, z.B. nach einer geeigneten Modifikation der Verfahrensvariante (a) erhalten werden, wobei das Ausgangsmaterial der Formel III gegebenenfalls in situ gebildet wird. So kann man z.B. ein 1-$R_2$-2-Ac-Vinyl-N-$R_1$-amin, wie einen 3-Amino-crotonsäure-niederalkylester, einen 4-$Z_2$-Acetessigsäureester, wie ein 4-Chlor-acetessigsäureniederalkylester, und einen R-Carboxaldehyd miteinander umsetzen und, ohne Isolieren eines Zwischenprodukts der Formel III, direkt zur gewünschten Verbindung der Formel I gelangen.

Die Verfahrensvariante (c) wird in an sich bekannter Weise durchgeführt. Ein Derivat des Aldehyds der Formel IV kann eines der obgenannten, z.B. die entsprechende Dihalogenmethyl-Verbindung sein. Die Reaktion wird in Ab-, vorzugsweise in Anwesenheit eines Lösungs- oder Verdünnungsmittels oder eines entsprechenden Gemisches und/oder eines Kondensationsmittels durchgeführt, wobei man unter Kühlen, bei Raumtemperatur, oder vorzugsweise unter Erwärmen, z.B. in einem Temperaturbereich von etwa 0°C bis etwa 200°C, vorzugsweise von etwa 40°C bis etwa 150°C, und, falls notwendig, in einem geschlossenen Gefäss, gegebenenfalls unter Druck, und/oder unter einer Inertgasatmosphäre arbeitet.

Das Ausgangsmaterial der Formel V kann in an sich bekannter Weise hergestellt werden; z.B. kann man durch Reaktion eines 1-$R_2$-2-Ac-Vinyl-N-$R_1$amins mit einer geeigneten 4-$Z_2$-Acetessigsäure oder vorzugsweise einem Derivat, wie einem Niederalkylester davon, worin $Z_2$ z.B. für Halogen, insbesondere Chlor, steht, direkt zu einem Ausgangsmaterial der Formel V gelangen oder dieses durch Ringschluss eines intermediär erhältlichen N-$R_1$-N-(1-$R_2$-2-Ac-Vinyl)-N-[1-($Z_1$-methylen)-2-$Z_2$-äthyl]-amins bilden.

Ausgangsstoffe der Formel VI können entsprechend dem darin enthaltenen Rest $Ac_o$ beispielsweise Carbonsäuren ($Ac_o$ ist Carboxyl), Carbonsäureanhydride, insbesondere gemischte Anhydride, wie Säurehalogenide, z.B. -chloride oder -bromide ($Ac_o$ ist Halogencarbonyl, z.B. Chlor- oder Bromcarbonyl), weiter aktivierte Ester, z.B. Cyanomethyl- oder Pentachlorphenylester ($Ac_o$ ist Cyanmethoxy- oder Pentachlorphenyloxycarbonyl) sein. Diese können, gegebenenfalls in Gegenwart von Kondensationsmitteln, durch Behandeln mit einem Alkohol, wie einem unsubstituierten oder substituierten Niederalkanol, oder einem reaktionsfähigen Derivat davon, z.B. einem entsprechenden Alkoholat, freie Carbonsäuren auch durch Umsetzen mit einer geeigneten Diazo-Verbindung, wie einem unsubstituierten oder substituierten Diazoniederalkan, in Verbindungen der Formel I umgewandelt werden, in denen Ac den Acylrest eines Monoesters der Kohlensäure darstellt. Solche Verbindungen können ebenfalls erhalten werden, wenn als Ausgangsstoffe Salze, insbesondere Alkalimetall- oder Erdalkalimetallsalze der entsprechenden freien Carbonsäuren verwendet, und diese mit reaktionsfähigen Estern von Alkoholen, wie unsubstituierten oder substituierten Niederalkanolen, wie entsprechenden Halogeniden, z.B. Chloriden, Bromiden oder Iodiden, oder organischen Sulfonsäureestern, z.B. Niederalkansulfonsäure- oder Arensulfonsäureestern, wie Methansulfonsäure- bzw. p-Toluolsulfonsäureestern, behandelt, oder wenn man entsprechende hydrolysierbare Iminoester, wie Iminoniederalkylester, zu den Estern hydrolysiert.

Die Umsetzung von freien Carbonsäuren mit Alkoholen, wie unsubstituierten oder substituierten Niederalkanolen, erfolgt vorteilhaft in Gegenwart eines sauren, wasserabspaltenden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, z.B. in inerten organischen Lösungsmitteln durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B.

Alkalimetall- oder Erdalkalimetallhydroxiden oder -carbonaten, wie Natrium-, Kalium- oder Calciumhydroxid bzw. -carbonat, mit Alkoholen oder mit Alkoholaten, z.B. Alkalimetall-niederalkanolaten, umgesetzt.

Die Umsetzung von reaktionsfähigen Estern, z.B. Cyanmethyl- oder Pentachlorphenylestern, mit Alkoholen werden beispielsweise in einem, gegenüber den Reaktionsteilnehmern inerten Lösungsmittel, z.B. im Temperaturbereich von etwa 0°C bis etwa 120°C, vorzugsweise bei Raumtemperatur bis etwa 60°C, durchgeführt.

Eine Gruppe $Ac_o$ kann z.B. auch eine verätherte Hydroxy-formimidoylgruppe, wie Niederalkoxy-formimidoyl, z.B. Aethoxyformimidoyl, darstellen. Die Hydrolyse eines entsprechenden Imidoester-, insbesondere Imidoniederalkylesterausgangsstoffs erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann ferner z.B. aus einem Gemisch von Nitril, Alkohol und Schwefelsäure mit geeignetem Wassergehalt, ohne Isolierung des *in situ* entstandenen Imidoesters, die gewünschte Esterverbindung der Formel I erhalten.

Verbindungen der Formel I, in denen Ac den Acylrest einen Kohlensäuremonoamids darstellt, kann man aus Verbindungen der Formel VI erhalten, in denen $Ac_o$ für Carboxylgruppen, Säureanhydridgruppen, wie Halogencarbonyl, z.B. Chlorcarbonyl, oder aktivierte Estergruppen, wie Cyanmethoxy- oder Pentachlorphenyloxycarbonyl, stehen, indem man solche Ausgangsstoffe, gegebenenfalls in Gegenwart eines geeigneten Kondensationsmittels, mit Ammoniak oder einem Ammoniak abgebenden Mittel oder einem N-mono- oder N,N-disubstituierten Amin umsetzt.

Diese Umwandlungen von Carboxy- und geeignet funktionell abgewandelten reaktionsfähigen Carboxygruppen in entsprechende Carbamoylgruppen werden in an sich bekannter Weise, z.B. nach den für die Bildung von Estergruppen beschriebenen Verfahren durchgeführt.

Ausgangsstoffe der Formel VI mit freier Carboxylgruppe $Ac_o$ können z.B. erhalten werden, indem man den entsprechenden 2-Cyanäthylester herstellt, wobei man z.B. in einem der vorstehend beschriebenen Verfahren ab) oder ag) eine Verbindung der Formel XI einsetzt, in der Ac eine 2-Cyanoäthoxycarbonylgruppe ist; z.B. kann man einen gegebenenfalls in der Aminogruppe entsprechend der Definition für $R_1$ substituierten 3-Amino-crotonsäure-2-cyan-äthylester mit den übrigen Reaktionskomponenten umsetzen, und anschliessend die so erhältliche 2-Cyanoäthylester-Verbindung unter milden Bedingungen, z.B. mittels wässrigem oder wässerig-niederalkanolischem 1-n. Natriumhydroxid bei Raumtemperatur, zur freien Carbonsäure spalten. Letztere kann, falls notwendig, in an sich bekannter Weise in die gewünschten reaktionsfähigen funktionellen Derivate übergeführt werden.

Verfahrensgemäss erhältliche Verbindungen der Formel I können in an sich bekannter Weise in andere Verbindungen der Formel I umgewandelt werden.

So kann man z.B. in Verbindungen der Formel I, worin $R_1$ für Wasserstoff steht, durch Behandeln mit einem reaktionsfähigen Ester eines Alkohols der Formel $R_1$-OH (XXIII) einen organischen Rest $R_1$ einführen, und so zu Verbindungen der Formel I gelangen, worin $R_1$ von Wasserstoff verschieden ist.

Reaktionsfähige Ester von Verbindungen der Formel XXIII, z.B. von unsubstituierten oder substituierten Niederalkanolen, sind solche mit starken, anorganischen oder organischen Säuren; dabei kommen beispielsweise die entsprechenden Halogenide, insbesondere Chloride, Bromide oder Jodide, ferner Sulfate, weiter Niederalkansulfonsäure- oder Arensulfonsäureester, z.B. Methansulfonsäure-, Benzolsulfonsäure- oder p-Toluolsulfonsäureester, in Betracht. Die Umsetzung wird, falls notwendig, unter Kühlen oder Erwärmen, z.B. in einem Temperaturbereich von etwa 0°C bis etwa 100°C, in Gegenwart eines geeigneten basischen Kondensationsmittels, z.B. eines Alkalimetalls, Alkalimetallamids oder -hydrids, oder eines Alkalimetallniederalkoholats, wie Natrium- oder Kalium-methanolat, -äthanolat oder tert.-butanolat, in An- oder Abwesenheit eines Lösungs- oder Verdünnungsmittels, bei erniedrigter oder erhöhter Temperatur, z.B. in einem Temperaturbereich von etwa 0°C bis etwa 100°C, und/oder unter atmosphärischem Druck oder in einem geschlossenen Gefäss durchgeführt.

Vorzugsweise verwendet man in solchen N-Substituierungsreaktionen in erster Linie Verbindungen der Formel I, die keine Hydroxy- und/oder andere Aminogruppen als Substituenten aufweisen, da diese unter Umständen ebenfalls mit dem reaktionsfähigen Ester eines Alkohols der Formel (XXIII) in Reaktion treten können.

Ferner können in verfahrensgemäss erhältlichen Verbindungen der Formel I vorhandene Substituenten in andere Substituenten umgewandelt werden.

So kann man z.B. veresterte Carboxygruppen, wie entsprechende Gruppen Ac und/oder $R_2$ oder entsprechende Substituenten eines Restes $R_2$, durch Umesterung in andere Ester überführen. Dabei verwendet man vorzugsweise entsprechende Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Hydroxyverbindung und/oder einem inerten organischen, vorzugsweise ebenfalls deutlich über dem Alkohol der veresterten Gruppe siedenden Lösungsmittel, vorzugsweise in Gegenwart eines Katalysators, z.B. eines Alkalimetall-niederalkanolats, wie Natrium- oder Kalium-methanolat oder -äthanolat, in der Wärme und üblicherweise unter Abdestillieren des freigesetzten Alkohols durch.

Verbindungen der Formel I mit veresterten Carboxygruppen, wie Niederalkoxycarbonylgruppen, insbesondere eine entsprechende Gruppe Ac, können in solche mit entsprechenden Carboxamidgruppen umgewandelt werden, z.B. durch Behandeln mit Ammoniak, ferner mono- oder disubstituierten Aminen, wenn notwendig, unter erhöhter Temperatur und/oder in einem geschlossenen Gefäss.

Verbindungen der Formel I, in welchen die Gruppen Ac für Carbamoyl steht, kann man ausgehend von Verbindungen der Formel I, in welchen Ac Cyan darstellt, z.B. hydrolytisch, vorzugsweise unter sauren oder basischen Bedingungen, z.B. in Gegenwart eines Alkalimetall-, wie Natriumhydroxids, und, wenn erwünscht, von Wasserstoffperoxid in einem wässrig-alkoholischen Lösungsmittel, wie wässrigem Aethanol, erhalten.

In einer Verbindung der Formel I, worin $R_2$ Hydroxy-niederalkyl, z.B. Hydroxymethyl, darstellt, kann Hydroxy durch Behandeln mit einem Halogenierungsmittel, wie einem geeigneten anorganischen oder organischen Säurehalogenid z.B. Thionylchlorid, in Halogen, wie Chlor, umgewandelt werden.

Eine Verbindung der Formel I, worin $R_2$ Halogenniederalkyl, z.B. Chlormethyl, bedeutet, kann man mit einer geeigneten Cyanverbindung, wie einem Metall-, z.B. Alkalimetallcyanid, oder einem Ammoniumcyanid behandeln und so Verbindungen der Formel I erhalten, worin $R_2$ Cyanniederalkyl, z.B. Cyanmethyl, darstellt.

In einer Verbindung der Formel I, worin $R_2$ Cyan oder Cyanniederalkyl darstellt, kann z.B. durch Behandeln mit einem Alkohol, wie Niederalkanol, in Gegenwart einer Säure, wie einer Mineralsäure,

z.B. Schwefelsäure, und von Wasser die Cyangruppe in eine veresterte Carboxylgruppe, wie Niederalkoxycarbonyl, umgewandelt werden.

Ferner kann man Verbindungen der Formel I, worin $R_2$ für eine 2-Aminoniederalkylgruppe, insbesondere eine 2-N,N-disubstuierte Aminoäthylgruppe steht, auch dadurch erhalten, dass man eine Verbindung der Formel I, worin $R_2$ für Niederalkyl, insbesondere Methyl steht, mit Formaldehyd oder einem solchen abgebenden Mittel, wie Paraformaldehyd, und einem Amin, insbesondere einen N,N-disubstituierten Amin, nach dem Mannich-Verfahren umsetzt.

Ferner kann man Verbindungen der Formel I, worin $R_2$ ein am gleichen Kohlenstoffatom zwei Niederalkoxy, z.B. Methoxy oder Aethoxy, enthaltendes Niederalkyl, insbesondere Diniederalkoxymethyl, z.B. Dimethoxymethyl oder Diäthoxymethyl, darstellt, in Verbindungen der Formel I umwandeln, worin $R_2$ ein Oxo enthaltendes Niederalkyl, insbesondere Formyl, bedeutet. Die Umwandlung der Ketal- bzw. Acetalgruppierung in die freie Carbonylgruppe kann in an sich bekannter Weise, z.B. durch Behandeln mit einem sauren Reagens, wie einer Säure, insbesondere einer Mineralsäure, z.B. Chlorwasserstoffsäure, oder einer organischen Sulfonsäure, z.B. p-Toluolsulfonsäure, durchgeführt werden.

Eine Verbindung der Formel I, worin $R_2$ ein Oxo enthaltendes Niederalkyl, insbesondere Formyl, bedeutet, kann durch Reduktion, z.B. Behandeln mit einem geeigneten Hydridreduktionsmittel, wie einem Alkalimetallborhydrid, z.B. Natriumborhydrid, in eine Verbindung der Formel I umgewandelt werden, worin $R_2$ für Hydroxyniederalkyl, insbesondere Hydroxymethyl, steht. Ferner kann man sie durch Behandeln mit einem gegebenenfalls O-substituierten Hydroxylamin, wie Niederalkoxyamin, oder einem Säureadditionssalz davon, in eine Verbindung der Formel I umwandeln, worin $R_2$ ein gegebenenfalls O-substituiertes Hydroxyimino enthaltendes Niederalkyl, insbesondere gegebenenfalls O-substituierte, wie O-niederalkyliertes Hydroxyiminomethyl, darstellt.

In einer Verbindung der Formel I, worin $R_2$ ein gegebenenfalls O-substituiertes Hydroxyimino enthaltendes Niederalkyl darstellt, kann dieses durch Reduktion, z.B. durch Behandeln mit einem geeigneten Hydridreduktionsmittel, wie einem Alkalimetallborhydrid, z.B. Natriumcyanborhydrid, in ein gegebenenfalls N-monosubstituiertes Amino enthaltendes Niederalkyl umgewandelt werden. Eine Verbindung der Formel I mit einer Hydroxyiminoniederalkyl-, insbesondere Hydroxyiminomethylgruppe als $R_2$ kann durch Dehydratisieren, z.B. durch Behandeln mit einem anorganischen Säurehalogenid, wie Phosphoroxychlorid, oder einer Carbodiimidverbindung, wie N,N'-Dicyclohexyl-carbodiimid, in eine Verbindung der Formel I umgewandelt werden, worin $R_2$ Cyan oder Cyan-niederalkyl darstellt.

Im Zusammenhang mit den obigen Verfahren, insbesondere mit der Verfahrensvariante (d) und den Nachoperationen, muss darauf geachtet werden, dass die Reaktionsbedingungen jeweils so gewählt werden, dass der Lactonring der 5-Oxo-1,4,5,7-tetrahydrofuro[3,4-b]pyridin-Grundstruktur an der Reaktion nicht teilnimmt und z.B. aufgespalten wird.

Je nach Reaktionsbedingungen können Verbindungen der Formel I mit salzbildenden, insbesondere basischen Eigenschaften in freier Form oder in Form von Salzen erhalten werden.

So können erhaltene Säureadditionssalze in an sich bekannter Weise, z.B. durch Behandeln mit einer Base, wie einem Alkalimetallhydroxid, in die freien Verbindungen oder z.B. durch Behandeln mit geeigneten Säuren oder Derivaten davon in andere Salze umgewandelt werden. Erhaltene freie Verbindungen der Formel I mit salzbildenden basischen Eigenschaften können, z.B. durch Behandeln mit Säuren oder entsprechenden Anionenaustauschern, in ihre Salze umgewandelt werden.

Infolge der engen Beziehung zwischen den salzbildenden Verbindungen der Formel I in freier Form und in Form von Salzen, sind im Vorausgegangenen und nachfolgend unter den freien Verbindungen oder ihren Salzen sinn- und zweckmässig gegebenenfalls auch die entsprechenden Salze bzw. freien Verbindungen zu verstehen.

Die Verbindungen der Formel I, einschliesslich Salze von entsprechenden salzbildenden Verbindungen, können auch in Form ihrer Hydrate erhalten werden, oder ihre Kristalle können z.B. das zur Kristallisation verwendete Lösungsmittel einschliessen.

Die Verbindungen der Formel I können, je nach Verfahrensreaktion und/oder Art der Ausgangsstoffe, in Form von Racematgemischen, Racematen oder optischen Antipoden erhalten werden.

Erhaltene Racematgemische können auf Grund der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salzbildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren d) unter Verwendung einer optisch aktiven Säure der Formel VI ($Ac_o$ bedeutet Carboxy) erhalten, wobei man diese

z.B. aus der entsprechenden racemischen Säure in üblicher Weise, z.B. durch Salzbildung mit einer optisch aktiven Base, Trennung der diastereomeren Salze und Freisetzung der optisch aktiven Säure, oder unter Verwenden eines reaktionsfähigen funktionellen Derivats einer optisch aktiven Säure bilden kann.

Ferner kann man z.B. Verbindungen der Formel I, die eine veresterte Carboxygruppe, z.B. eine entsprechende Gruppe Ac, aufweisen, unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Diastereomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, und/oder seiner Racemate bzw. Antipoden verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der vorliegenden Erfindung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, in erster Linie als cardiotonisch-wirksame und/oder blutdrucksteigernde, das Zentralnervensystem aktivierende, insbesondere stimulierende, ferner Blutzucker-senkende Verbindungen. Dabei kann man sie, vorzugsweise in Form von pharmazeutischen Präparaten, in einem Verfahren zur prophylaktischen und/oder therapeutischen Behandlung des tierischen oder menschlichen Körpers, insbesondere zur Behandlung von Zuständen mit verminderter Pumpleistung des Herzens und mit arterieller Hypotonie und von anderen entsprechenden cardiovasculären Krankheiten, ferner zur Behandlung retardierter Depressionen verschiedenen Schweregrades, von psychischen, von fehlendem Antrieb und Initiative begleitenten Störungen, sowie von hypokinetischen, geriatrischen Patienten, ferner auch zur Senkung des Blutzuckers verwenden.

Die Dosierung des Wirkstoffs, der allein oder zusammen mit dem üblichen Träger- und Hilfsmaterial verabreicht wird, hängt von der zu behandelnden Spezies, deren Alter und individuellen Zustand, sowie der Verabreichungsweise ab. Die Einzeldosen liegen z.B. für Mammalien mit einem Körpergewicht von etwa 70 kg, je nach Art der Erkrankung, individuellem Zustand und Alter, vorzugsweise zwischen etwa 10 und 300 mg, z.B. zwischen etwa 50 und etwa 250 mg, z.B. bei oraler Verabreichung.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 mg bis etwa 300 mg, insbesondere

von etwa 20 mg bis etwa 200 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen,

wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1: Ein Gemisch von 22,5 g 2-(2-Methoxy-äthoxy)-benzaldehyd, 16,2 g 3-Aminocrotonsäureäthylester und 23,5 g 4-Acetoxy-acetessigsäureäthylester in 400 ml absolutem Aethanol wird während 25 Stunden unter Rückfluss erhitzt. Dann werden 7,0 g Kaliumhydroxid zugegeben und das Gemisch weitere 3 Stunden unter Rückfluss erhitzt. Die Reaktionslösung wird unter vermindertem Druck eingedampft und der Rückstand zwischen Essigsäureäthylester und Wasser verteilt. Die organische Phase wird eingedampft und an einer Silicagel-Mitteldrucksäule unter Verwendung eines 1:1 Gemisches von Essigsäureäthylester und n-Hexan als Eluiermittel chromatographiert. Der so erhältliche 2-Methyl-4-[2-(2-methoxyäthoxy)-phenyl]-5-oxo-1,4,5,7-tetrahydro-furo[3,4-b]pyridin-3-carbonsäure-äthylester schmilzt nach Umkristallisieren aus Methanol bei 174-176°.

Das Ausgangsmaterial kann wie folgt erhalten werden:
Ein Gemisch von 36,6 g Salicylaldehyd, 165,6 g Kaliumcarbonat und 63,0 g 2-Methoxyäthylchlorid wird während 24 Stunden in 600 ml Dimethylformamid bei 60° gerührt. Die braune Suspension wird unter vermindertem Druck eingedampft, der Rückstand in Essigsäureäthylester suspendiert, abfiltriert und das Filtrat im Vakuum eingeengt. Das zurückbleibende Oel wird destilliert, Kp 85°/0,9 mm Hg, und stellt reinen 2-(2-Methoxy-äthoxy)-benzaldehyd dar.

Beispiel 2: Ein Gemisch von 30,0 g 2-(2-Methylthio-äthoxy)-benzaldehyd, 19,7 g 3-Aminocrotonsäureäthylester und 28,8 g 4-Acetoxy-acetessigsäure-äthylester in 500 ml absolutem Aethanol wird während 15 Stunden unter Rückfluss erhitzt. Die Reaktionslösung wird unter vermindertem Druck eingedampft und der Rückstand an der 40-fachen Menge Silicagel unter Verwendung eines 1:2 Gemisches von Essigsäureäthylester und n-Hexan als Eluiermittel chromatographiert. Der so erhaltene 2-Acetoxymethyl-6-methyl-4-[2-(2-methylthio-äthoxy)-phenyl]-1,4-dihydropyridin-3,5-dicarbonsäure-diäthylester wird in 30 ml Aethanol mit 0,4 g Kaliumhydroxid 1 Stunde unter Rückfluss erhitzt. Nach Eindampfen unter vermindertem Druck und Verteilen des Rückstandes zwischen Essigsäureäthylester und Wasser wird die organische Phase eingedampft und an einer Silicagel-Mitteldrucksäule unter Verwendung eines 1:1 Gemisches von Essigsäureäthylester und n-Hexan als Eluiermittel chromatographiert. Der so erhältliche 2-Methyl-4-[2-(2-methylthioäthoxy)-phenyl]-5-oxo-1,4,5,7-tetrahydro-furo[3,4-b]pyridin-3-carbonsäure-äthylester schmilzt nach Umkristallisieren aus Methanol bei 154-156°.

Das Ausgangsmaterial kann wie folgt erhalten werden:
Ein Gemisch von 36,6 g Salicylaldehyd, 165,6 g Kaliumcarbonat und 77,4 g 2-Chloräthyl-methylsulfid in 600 ml Dimethylformamid wird während 24 Stunden bei 60° gerührt. Die braune Suspension wird unter vermindertem Druck eingedampft, der Rückstand in Essigsäureäthylester suspendiert, abfiltriert und das Filtrat im Vakuum eingeengt. Das zurückbleibende Oel wird destilliert, Kp. 95°/1,0 mm Hg, und stellt den reinen 2-(2-Methylthio-äthoxy)-benzaldehyd dar.

Beispiel 3: Ein Gemisch von 29,1 g 2-Difluormethoxy-benzaldehyd, 21,8 g 3-Amino-crotonsäureäthylester und 18,8 g 4-Chlor-acetessigsäureäthylester in 70 ml absolutem Aethanol wird während 72 Stunden unter Rühren auf 60° erhitzt. Die Reaktionslösung wird abgekühlt und die ausgefallenen Kristalle abfiltriert und in einer grossen Menge Essigsäureäthylester gelöst. Die Lösung wird mehrmals mit Wasser gewaschen, dann über Natriumsulfat getrocknet und unter vermindertem Druck eingedampft. Der 4-(2-Difluormethoxy-phenyl)-2-methyl-5-oxo-1,4-5,7-tetrahydro-furo[3,4-b]pyridin-3-carbonsäureäthylester wird aus Essigsäureäthylester umkristallisiert und schmilzt bei 174-175°.

Das Ausgangsmaterial kann wie folgt erhalten werden: Ein Gemisch von 157,4 g Salicylaldehyd, 646 ml 30%-iger wässriger Natriumhydroxidlösung, 320 ml Wasser und 720 ml Dioxan wird unter Rühren auf 78° erwärmt und während 2 Stunden 305,3 g Chlordifluormethan eingeleitet. Die Reaktionslösung wird abgekühlt, auf Eis ausgegossen und mit Diäthyläther extrahiert, und die organische Phase über Natriumsulfat getrocknet und unter vermindertem Druck eingedampft. Der als oranges Oel erhaltene 2-Difluormethoxy-benzaldehyd wird als Rohprodukt weiter verarbeitet.

Beispiel 4: Analog dem im Beispiel 3 beschriebenen Verfahren erhält man durch Erhitzen unter Rückfluss während 22 Stunden aus einem Gemisch von 21,0 g 4-Difluormethoxy-benzaldehyd, 11,1 g 3-Amino-crotonsäureäthylester und 4-Chloracetessigsäureäthylester in 100 ml absolutem Aethanol den 4-(4-Difluormethoxy-phenyl)-2-methyl-5-oxo-1,4,5,7-tetrahydro-furo[3,4-b]pyridin-3-carbonsäureäthylester, der nach Umkristallisieren aus einem Gemisch von Essigsäureäthylester und Petroläther bei 167-168° schmilzt.

Der als Ausgangsmaterial verwendete 4-Difluormethoxy-benzaldehyd kann nach dem im Beispiel 3 beschriebenen Verfahren durch Behandeln von 4-Hydroxy-benzaldehyd mit Chlordifluormethan in Gegenwart von Natriumhydroxid hergestellt werden.

Beispiel 5: Analog dem im Beispiel 3 beschriebenen Verfahren erhält man durch Erhitzen unter Rückfluss während 8 Stunden aus einem Gemisch von 36,0 g 2-(2-Chlor-1,1,2-trifluor-äthoxy)-benzaldehyd, 19,5 g 3-Amino-crotonsäureäthylester und 32,9 g 4-Chlor-acetessigsäureäthylester in 200 ml absolutem Aethanol den 4-[2-(2-Chlor-1,1,2-trifluor-äthoxy)-phenyl]-2-methyl-5-oxo-1,4,5,7-tetrahydro-furo-[3,4-b]pyridin-3-carbonsäureäthylester, der nach Umkristallisieren aus einem Gemisch von Aethanol und Diäthyläther bei 171-173° schmilzt.

Das Ausgangsmaterial kann wie folgt erhalten werden:
Ein Gemisch von 61,0 g Salicylaldehyd, 126,75 g Chlortrifluoräthylen, 32,5 g Kaliumhydroxyd und 370 ml Dimethylformamid wird im Autoklaven während 12 Stunden auf 60° erhitzt. Die Reaktionslösung wird auf 2000 ml Eiswasser ausgegossen und erschöpfend mit Diäthyläther extrahiert; die organische Phase wird mit Wasser gewaschen, über Natriumsulfat getrocknet und unter vermindertem Druck eingedampft. Der als rotes Oel zurückbleibende 2-(2-Chlor-1,1,2-trifluor-äthoxy)-benzaldehyd wird roh weiter verarbeitet.

Beispiel 6: Ein Gemisch von 1,72 g 2-Difluormethoxy—benzaldehyd, 1,3 g 3-Amino-crotonsäureäthylester und 1,0 g Tetronsäure (Tetrahydrofuran-2,4-dion) in 20 ml absolutem Aethanol wird während 2 Stunden auf 60° erhitzt. Die Reaktionslösung wird unter vermindertem Druck eingedampft, und der Rückstand an der 50-fachen Menge Silikagel (Elution mit einem 6:4-Gemisch von Toluol und Essigsäureäthylester) chromatographiert. Der so erhältliche 4-(2-Difluormethoxy-phenyl)-2-methyl-5-oxo-1,4,5,7-tetrahydro-furo[3,4-b]pyridin-3-carbonsäure-äthylester schmilzt nach Umkristallisieren aus Essigsäureäthylester bei 174-175°.

Beispiel 7: Ein Gemisch von 17,2 g 2-Difluormethoxy-benzaldehyd, 8,2 g 3-Amino-crotonsäurenitril und 11,1 g 4-Chlor-acetessigsäure-äthylester in 200 ml absolutem Aethanol wird während 48 Stunden unter Rückfluss erhitzt. Die Reaktionslösung wird unter vermindertem Druck eingedampft und der Rückstand zwischen Essigsäureäthylester und Wasser verteilt. Die organische Phase wird mit Natriumsulfat getrocknet, eingedampft und der Rückstand an der 30-fachen Menge Silicagel unter Verwendung eines 1:1 Gemisches von Toluol und Essigsäureäthylester als Eluiermittel chromatographiert. Das so erhaltene 4-(2-Difluormethoxy-phenyl)-2-methyl-5-oxo-1,4,5,7-tetrahydro-furo[3,4-b]pyridin-3-carbonitril schmilzt nach Umkristallisieren aus einem Gemisch von Methanol und Methylenchlorid bei 228-232°.

Beispiel 8: Analog dem im Beispiel 3 beschriebenen Verfahren erhält man aus einem Gemisch von 7,9 g 2-Difluormethoxybenzaldehyd; 7,3 g 3-Amino-crotonsäure-(2-methoxyäthyl)-ester und 7,57 g 4-Chlor-acetessigsäureäthylester in 80 ml absolutem Aethanol in 42 Stunden unter Rückfluss den 4-(2-Difluormethoxyphenyl)-2-methyl-5-oxo-1,4,5,7-tetrahydro-furo[3,4-b]pyridin-3-carbonsäure-(2-methoxyäthyl)-ester, der nach Umkristalliseren aus einem Gemisch von Chloroform und Methanol bei 165-167° schmilzt.

Beispiel 9: Analog dem im Beispiel 3 beschriebenen Verfahren erhält man aus einem Gemisch von 7,9 g 2-Difluormethoxybenzaldehyd, 5,3 g 3-Amino-crotonsäuremethylester und 7,57 g 4-Chlor-acetessigsäureäthylester in 80 ml absolutem Aethanol in 42 Stunden unter Rückfluss den 4-(2-Difluormethoxyphenyl)-2-methyl-5-oxo-1,4,5,7-tetrahydro-furo[3,4-b]pyridin-3-carbonsäure-methylester, der nach Umkristallisation aus einem Gemisch von Chloroform, Methanol und Petroläther bei 200-202° schmilzt.

Beispiel 10: Analog dem im Beispiel 3 beschriebenen Verfahren erhält man aus einem Gemisch von 17,2 g Difluormethoxybenzaldehyd, 14,3 g 3-Amino-crotonsäure-isopropylester und 16,5 g 4-Chlor-acetessigsäureäthylester in 150 ml absolutem Aethanol in 42 Stunden unter Rückfluss den 4-(2-Difluormethoxyphenyl)-2-methyl-5-oxo-1,4,5,7-tetrahydro-furo[3,4-b]pyridin-3-carbonsäure-isopropylester, der nach Umkristallisieren aus einem Gemisch von Chloroform und Methanol bei 82-88° chmilzt.

Beispiel 11: Analog dem im Beispiel 3 beschriebenen Verfahren erhält man aus einem Gemisch von 7,9 g 3-Difluormethoxybenzaldehyd,,5,9 g 3-Amino-crotonsäureäthylester und 7,57 g 4-Chlor-acetessigsäureäthylester in 80 ml absolutem Aethanol in 46 Stunden unter Rückfluss

den 4-(3-Difluormethoxyphenyl)-2-methyl-5-oxo-1,4,5,7-tetrahydro-furo [3,4-b]pyridin-3-carbonsäure-äthylester, der nach Umkristallisieren aus einem Gemisch von Chloroform, Methanol und Aether bei 164-166° schmilzt.

Beispiel 12: Analog dem im Beispiel 3 beschriebenen Verfahren erhält man aus einem Gemisch von 10,0 g 2,3-Di(difluormethoxy)-benzaldehyd, 5,4 g 3-Amino-crotonsäureäthylester und 6,9 g 4-Chlor-acetessigsäure-äthylester in 10 ml absolutem Aethanol in 42 Stunden unter Rückfluss den 4-[2,3-Di-(difluormethoxy)-phenyl]-2-methyl-5-oxo-1,4,5,7-tetrahydro-furo[3,4-b]pyridin-3-carbonsäure-äthylester, der nach Umkristallisation aus einem Gemisch von Chloroform, Methanol und Petroläther bei 203-205° schmilzt.

Beispiel 13: Analog dem im Beispiel 3 beschriebenen Verfahren erhält man aus einem Gemisch von 8,8 g 2-Difluormethylmercaptobenzaldehyd, 6,1 g 3-Amino-crotonsäureäthylester und 7,75 g 4-Chlor-acetessigsäureäthylester in 100 ml absolutem Aethanol in 45 Stunden unter Rückfluss den 4-(2-Difluormethylmercaptophenyl)-2-methyl-5-oxo-1,4,5,7-tetrahydro-furo[3,4-b]pyridin-3-carbonsäure-äthylester, der nach Umkristallisation aus einem Gemisch von Chloroform, Methanol und Petroläther bei 238-239° schmilzt.

Beispiel 14: Tabletten, enthaltend 100 mg 4-(2-Difluormethoxy-phenyl)-2-methyl-5-oxo-1,4,5,7-tetrahydro-furo[3,4-b]pyridin-3-carbonsäure-äthylester, können wie folgt hergestellt werden:

Zusammensetzung (für 1000 Tabletten)

| | |
|---|---|
| 4-(2-Difluromethoxy-phenyl)-2-methyl-5-oxo-1,4,5,7-tetrahydro-furo[3,4-b]pyridin-3-carbonsäure-äthylester | 100.00 g |
| Lactose, gemahlen | 80.00 g |
| Maisstärke | 41.00 g |
| Polyvinylpyrrolidon | 5.00 g |
| Cellulose, mikrokristallin | 20.00 g |
| Siliciumdioxid | 2.50 g |
| Magnesiumstearat | 1.50 g |
| Aethanol | q.s |

Der 4-(2-Difluormethoxy-phenyl)-2-methyl-5-oxo-1,4,5,7-tetrahydro-furo[3,4-b]pyridin-3-carbonsäure-äthylester, die Lactose und die Maisstärke werden zusammen gemischt, und das Gemisch mit einer äthanolischen Lösung des Polyvinylpyrrolidons befeuchtet; das feuchte Gemisch wird geknetet, granuliert und getrocknet. Das trockene Granulat wird mit der Cellulose, dem Siliciumdioxid und dem Magnesiumstearat vermischt und durch ein Sieb (Maschenweite: 1,2 mm) getrieben. Das Siebgut wird erneut gemischt und zu Tabletten (Gewicht: 250 mg, Durchmesser: 9 mm) mit Bruchrille verpresst.

Beispiel 15: Tabletten, enthaltend 100 mg 4-(2-Difluormethoxy-phenyl)-2-methyl-5-oxo-1,4,5,7-tetrahydro-furo[3,4-b]pyridin-3-carbonsäure-äthylester, können wie folgt hergestellt werden:

Zusammensetzung (1000 Tabletten)

| | |
|---|---|
| 4-(2-Difluormethoxy-phenyl)-2-methyl-5-oxo-1,4,5,7-tetrahydro-furo[3,4-b]pyridin-3-carbonsäure-äthylester, | 100.00 g |
| Cellulose, mikrokristallin | 82.00 g |
| Natrium-carboxymethylcellulose | 14.50 g |
| Siliciumdioxid | 2.00 g |
| Magnesiumstearat | 1.50 g |

Der Wirkstoff und die Hilfsmittel werden zusammen vermischt und zu Tabletten (Gewicht: 200 mg; Durchmesser: 8 mm) mit Bruchrille verpresst.

Beispiel 16: Anstelle der in den Beispielen 14 und 15 als Wirkstoff verwendeten Verbindung können auch folgende Verbindungen der Formel I oder, sofern sie salzbildende Eigenschaften aufweisen, deren pharmazeutisch, verwendbare Salze, als Wirkstoffe in Tabletten, Dragées, Kapseln, Ampullenlösungen etc. verwendet werden:

4-(4-Difluormethoxy-phenyl)-2-methyl-5-oxo-1,4,5,7-tetrahydro-furo-[3,4-b]pyridin-3-carbonsäure-äthylester, 4-[2-(2-Chlor-1,1,2-trifluoräthoxy)-phenyl]-2-methyl-5-oxo-1,4,5,7-tetrahydrofuro[3,4-b]pyridin-3-carbonsäureäthylester, 4-(2-Difluormethoxy-phenyl)-2-methyl-5-oxo-1,4,5,7-tetrahydro-furo[3,4-b]pyridin-3-carbonitril, 4-(2-Difluormethoxyphenyl)-2-methyl-5-oxo-1,4,5,7-tetrahydro-furo[3,4-b]pyridin-3-carbonsäure-(2-methoxyäthyl)-ester, 4-(2-Difluormethoxyphenyl)-2-methyl-5-oxo-1,4,5,7-tetrahydro-furo[3,4-b]pyridin-3-carbonsäuremethylester, 4-(2-Difluormethoxyphenyl)-2-methyl-5-oxo-1,4,5,7-tetrahydro-furo[3,4-b]pyridin-3-carbonsäure-isopropylester, 4-(3-Difluormethoxyphenyl)-2-methyl-5-oxo-1,4,5,7-tetrahydro-furo[3,4-b]pyridin-3-carbonsäureäthylester, 4-[2,3-Di-(difluormethoxy)-phenyl]-2-methyl-5-oxo-1,4,5,7-tetrahydro-furo[3,4-b]pyridin-3-carbonsäure-äthylester, 4-(2-Difluormethylmercaptophenyl)-2-methyl-5-oxo-1,4,5,7-tetrahydro-furo[3,4-b]pyridin-3-carbonsäure-äthylester, 2-Methyl-4-[2-(2-methoxyäthoxy)-phenyl]-5-oxo-1,4,5,7-tetrahydro-furo[3,4-b]pyridin-3-carbonsäure-äthylester oder der 2-Methyl-4-[2-(2-methylthio-äthoxy)-phenyl]-5-oxo-1,4,5,7-tetrahydro-furo[3,4-b]pyridin-3-carbonsäure-äthylester.

Patentansprüche

1. 5-Oxo-1,4,5,7-tetrahydro-furo[3,4-b]pyridin-3-verbindungen der Formel

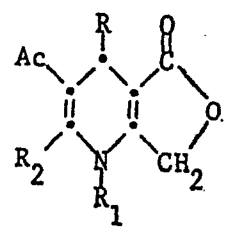

(I),

worin R einen durch Niederalkoxyniederalkoxy, Halogenniederalkoxy ausgenommen Trifluormethoxy, Niederalkylthioniederalkoxy oder Halogenniederalkylthio substituierten carbocyclischen oder heterocyclischen Arylrest bedeutet, $R_1$ Wasserstoff oder unsubstituiertes oder substituiertes Niederalkyl bedeutet, $R_2$ Wasserstoff, Niederalkyl oder durch freies, veräthertes oder verestertes Hydroxy, durch funktionell abgewandeltes Carboxy oder durch freies oder substituiertes Amino substituiertes Niederalkyl, funktionell abgewandeltes Carboxy oder freies oder substituiertes Amino bedeutet, wobei eine Aminogruppe $R_2$ mit einem Niederalkylrest $R_1$ verbunden sein kann, oder, falls $R_2$ für Niederalkyl steht, dieses zusammen mit dem benachbarten Acylrest Ac einen 2-Oxa-1-oxo-niederalkylenrest bilden kann, wobei dessen Carbonylgruppe mit dem 3-Ringkohlenstoffatom des Furo[3,4-b]pyridinrings verbunden ist, und Ac den Acylrest einer Säure darstellt, als Racematgemische, Racemate, optische Antipoden oder Salze, insbesondere pharmazeutisch verwendbare Salze von solchen Verbindungen mit salzbildenden Eigenschaften, in erster Linie Säureadditionssalze, wie pharmazeutisch verwendbare Säureadditionssalze von solchen Verbindungen mit salzbildenden basischen Eigenschaften.

2. Verbindungen der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzofuranyl, Benzothienyl, Chinolinyl oder Isochinolinyl bedeutet, wobei in diesen Resten Ringkohlenstoffatome durch Niederalkoxyniederalkoxy, Niederalkylthioniederalkoxy, Halogenniederalkoxy, ausgenommen Trifluormethoxy, oder Halogenniederalkylthio substituiert sind, und Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkylcarbamoyl oder Cyan als Substituenten enthalten kann, oder durch Hydroxy oder Oxido substituiert sein können, $R_1$ Wasserstoff, Niederalkyl, Diniederalkylamino-niederalkyl, Niederalkylenaminoniederalkyl, Morpholinoniederalkyl, Thiomorpholinoniederalkyl, Piperazinoniederalkyl oder 4-Niederalkyl-piperazinoniederalkyl darstellt, $R_2$ für Wasserstoff, Niederalkyl, Hydroxyniederalkyl, Niederalkoxyniederalkyl, Halogenniederalkyl, Niederalkoxycarbonylniederalkyl, Carbamoylniederalkyl, N-Niederalkylcarbamoylniederalkyl, N,N-Diniederalkylcarbamoylniederalkyl, Cyanniederalkyl, Aminoniederalkyl, Niederalkylaminoniederalkyl, Diniederalkylaminoniederalkyl, Niederalkylenaminoniederalkyl, Morpholinoniederalkyl, Thiomorpholinoniederalkyl, Piperazinoniederalkyl, 4-Niederalkylpiperazino-niederalkyl, Niederalkoxycarbonyl, Carbamoyl, N-Niederalkylcarbamoyl, N,N-Diniederalkylcarbamoyl, Cyan, Amino, Niederalkylamino, Diniederalkylamino-niederalkylamino, Niederalkylenaminoniederalkylamino, Morpholino-niederalkylamino, Diniederalkylamino, Niederalkylenamino, (2-Oxo-1-imidazolidinyl)-niederalkylenamino, Morpholino, Thiomorpholino, Piperazino, 4-Niederalkyl-piperazino, 4-Niederalkanoyl-piperazino, 4-Benzoyl-piperazino, 4-Furoylpiperazino oder 4-Thenoyl-piperazino steht, wobei eine Aminogruppe $R_2$ mit einem Niederalkylrest $R_1$ verbunden sein und mit diesem zusammen einen 1-Aza-niederalkylenrest bilden kann, dessen Stickstoffatom mit dem 2-Ringkohlenstoffatom des Furo[3,4-b]pyridinrings verbunden ist, oder, falls $R_2$ für Niederalkyl steht, dieses zusammen mit dem benachbarten Acylrest Ac einen 2-Oxa-1-oxo-niederalkylenrest bilden kann, dessen Carbonylgruppe mit dem 3-Ringkohlenstoffatom des Furo[3,4-b]-pyridinrings verbunden ist, und der Rest Ac für Niederalkanoyl, unsubstituiertes oder durch Niederalkyl, Niederalkoxy und/oder Halogen substituiertes Benzoyl, Niederalkylsulfonyl, unsubstituiertes oder durch Niederalkyl, Niederalkoxy und/oder Halogen substituiertes Phenylsulfonyl, Cyan, Niederalkoxycarbonyl,Hydroxyniederalkoxycarbonyl,Niederalkoxyniederalkoxycarbonyl,Aminoniederalkoxycarbonyl, Niederalkylaminoniederalkoxycarbonyl,Diniederalkylaminoniederalkoxycarbonyl, N-Niederalkyl-N-phenylniederalkyl-amino-niederalkoxycarbonyl, Niederalkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy und/oder Halogen substituiertes Phenylniederalkoxycarbonyl, Carbamoyl, N-Niederalkyl-carbamoyl, N,N-Diniederalkylcarbamoyl, N,N-Niederalkylencarbamoyl, Morpholinocarbonyl, Thiomorpholinocarbonyl, Piperazinocarbonyl oder 4-Niederalkyl-piperazinocarbonyl steht, als Racematgemische, Racemate, optische Antipoden oder Salze, insbesondere pharmazeutisch verwendbare Salze von solchen Verbindungen mit salzbildenden Eigenschaften, in erster Linie Säureadditionssalze, wie pharmazeutisch verwendbare Säureadditionssalze von solchen Verbindungen mit salzbildehden basischen Eigenschaften.

3. Verbindungen der Formel I, worin R für Phenyl, Naphthyl, Pyrryl, Furyl, Thienyl, Pyridyl oder über ein Ringkohlenstoff gebundenes Imidazolyl steht, die Niederalkoxyniederalkoxy, Niederalkylthioniederalkoxy, Halogenniederalkoxy, ausgenommen Trifluormethoxy, oder Halogenniederalkylthio als Substituenten enthalten, $R_1$ Wasserstoff, Niederalkyl, Diniederalkylamino-niederalkyl, Niederalkylenaminoniederalkyl oder (4-Morpholino)-niederalkyl darstellt, wobei Diniederalkylamino, Niederalkylenamino bzw. 4-Morpholino durch mindestens zwei Kohlenstoffatome vom Ringstickstoffatom getrennt sind, $R_2$ für Wasserstoff, Niederalkyl, Hydroxyniederalkyl, Halogenniederalkyl, Niederalkoxycarbonylniederalkyl, Cyanniederalkyl, Diniederalkylaminoniederalkyl, Niederalkoxycarbonyl, Cyan, Amino, (4-Morpholino)-niederalkylamino, ~~Niederalkylenamino~~, (2-Oxo-1-imidazolidinyl)-niederalkylenamino oder 4-(2-Furoyl)-piperazino bedeutet, wobei eine Aminogruppe $R_2$ mit einem Niederalkylrest $R_1$ verbunden sein und mit diesem zusammen einen 1-Aza-niederalkylenrest bilden kann, dessen Azastickstoffatom mit dem 2-Ringkohlenstoffatom des Furo[3,4-b]pyridinrings verbunden ist, oder, falls $R_2$ für Niederalkyl steht, dieses zusammen mit dem benachbarten Acylrest Ac einen 2-Oxa-1-oxo-niederalkylenrest bilden kann, dessen Carbonylgruppe mit dem 3-Ringkohlenstoffatom des Furo[3,4-b]pyridinrings verbunden ist, und der Rest Ac für Niederalkanoyl, Niederalkylsulfonyl, Cyan, Niederalkoxycarbonyl, Hydroxyniederalkoxycarbonyl, Niederalkoxyniederalkoxycarbonyl, N,N-Diniederalkylaminoniederalkoxycarbonyl, N-Niederalkyl-N-phenylniederalkyl-amino-niederalkoxycarbonyl, N,N-Niederalkylenaminoniederalkoxycarbonyl, (4-Morpholino)-niederalkoxycarbonyl, Carbamoyl, N-Niederalkyl-carbamoyl, N,N-Diniederalkyl-carbamoyl, N,N-Niederalkylencarbamoyl, 4-Niederalkyl-piperazino-carbonyl oder 4-Morpholinocarbonyl steht, als Racematgemische, Racemate, optische Antipoden oder Salze, insbesondere pharmazeutisch verwendbare Salze von solchen Verbindungen mit salzbildenden Eigenschaften, in erster Linie Säureadditionssalze, wie pharmazeutisch verwendbare Säureadditionssalze von solchen Verbindungen mit salzbildenden basischen Eigenschaften.

4. Verbindungen der Formel I, worin R für Phenyl, Pyridyl, z.B. 2-, 3- oder 4-Pyridyl, Furyl, z.B. 2-Furyl, oder Thienyl, z.B. 2-Thienyl steht, die durch Niederalkoxyniederalkoxy, z.B. Methoxymethoxy, oder 2-Methoxyäthoxy, Niederalkylthioniederalkoxy, z.B. 2-Methylthioäthoxy, Halogenniederalkoxy, ausgenommen Trifluormethoxy, z.B. Difluormethoxy oder 2-Chlor-1,1,2-trifluoräthoxy, oder Halogenniederalkylthio, z.B. Difluormethylmercapto, substituiert sind, wobei ein Phenylrest einen oder mehrere, gleiche oder verschiedene Substituenten aufweist, $R_1$ insbesondere Wasserstoff, ferner Niederalkyl, z.B. Methyl, 2-(Diniederalkylamino)-niederalkyl, besonders 2-Dimethylaminoäthyl, 2-(Niederalkylenamino)-niederalkyl, besonders 2-(Pyrrolidino)-äthyl oder 2-(Piperidino)-äthyl, oder 2-(4-Morpholino)-niederalkyl, besonders 2-(4-Morpholino)-äthyl, bedeutet, $R_2$ Niederalkyl, besonders Methyl, Hydroxyniederalkyl, besonders Hydroxymethyl, Halogenniederalkyl, insbesondere Chlormethyl, 2-(Diniederalkylamino)-niederalkyl, insbesondere 2-(Diniederalkylamino)-äthyl, Niederalkoxycarbonyl, besonders Methoxycarbonyl oder Aethoxycarbonyl, Cyan, Amino, (4-Morpholino)-niederalkylamino, besonders 2-(4-Morpholino)-äthylamino, Niederalkylenamino, besonders Pyrrolidino, (2-Oxo-1-imidazolidinyl)-niederalkylenamino, besonders 4-(2-Oxo-1-imidazolidinyl)-piperidino, oder 4-(2-Furoyl)-piperazino bedeutet, wobei eine Aminogruppe $R_2$ mit einem Niederalkylrest $R_1$ verbunden und zusammen mit diesem einen 1-Azaniederalkylenrest, insbesondere 1-Aza-1,3-propylen bilden kann, dessen Azastickstoffatom mit dem 2-Ringkohlenstoffatom des Furo[3,4-b]pyridinrings verbunden ist, oder falls $R_2$ für Niederalkyl, besonders Methyl steht, dieses zusammen mit dem benachbarten Acylrest Ac einen 2-Oxa-1-oxo-niederalkylenrest, besonders 2-Oxa-1-oxo-1,3-propylen bilden kann, dessen Carbonylgruppe mit dem 3-Ringkohlenstoffatom des Furo[3,4-b]pyridinrings verbunden ist, und der Rest Ac Niederalkanoyl, z.B. Acetyl, Niederalkylsulfonyl, z.B. Methylsulfonyl oder Aethylsulfonyl, Niederalkoxycarbonyl, besonders Methoxycarbonyl, Aethoxycarbonyl, n-Propyloxycarbonyl, Isopropyloxycarbonyl oder Isobutyloxycarbonyl, 2-Niederalkoxyniederalkoxycarbonyl, insbesondere

2-Methoxyäthoxy-carbonyl, 2- oder 3-Diniederalkylamino-niederalkoxycarbonyl, wie 2-Dimethylaminoäthoxycarbonyl, 2-Diäthylaminoäthoxycarbonyl oder 3-Dimethylaminopropyloxycarbonyl, 2- oder 3-(N-Niederalkyl-N-phenylniederalkyl-amino)-niederalkoxycarbonyl, z.B. 2-(N-Benzyl-N-methyl-amino)-äthoxycarbonyl, Carbamoyl, N-Niederalkyl-carbamoyl, z.B. N-Methylcarbamoyl, N,N-Diniederalkyl-carbamoyl, z.B. N,N-Dimethyl-carbamoyl, N,N-Niederalkylenamino-carbonyl, z.B. Pyrrolidino-carbonyl, 4-Niederalkylpiperazino-carbonyl, z.B. 4-Methyl-piperazino-carbonyl, oder 4-Morpholino-carbonyl oder Cyan bedeutet, als Racematgemische, Racemate, optische Antipoden oder Salze, insbesondere pharmazeutisch verwendbare Salze von solchen Verbindungen mit salzbildenden Eigenschaften, in erster Linie Säureadditionssalze, wie pharmazeutisch verwendbare Säureadditionssalze von solchen Verbindungen mit salzbildenden basischen Eigenschaften.

5. Verbindungen der Formel I, worin R für durch Niederalkoxyniederalkoxy, z.B. 2-Methoxyäthoxy, Niederalkylthioniederalkoxy, z.B. 2-Methylthioäthoxy, Halogenniederalkoxy, ausgenommen Trifluormethoxy, worin Halogen eine Atomnummer bis und mit 35 hat und insbesondere Fluor oder Chlor ist, z.B. Difluormethoxy oder 2-Chlor-1,1,2-difluor-äthoxy oder durch Halogenniederalkylthio, z.B. Difluormethylmercapto, substituiertes Phenyl steht, $R_1$ Wasserstoff bedeutet, $R_2$ Niederalkyl z.B. Methyl bedeutet, und der Rest Ac in erster Linie Niederalkoxycarbonyl, z.B. Methoxycarbonyl, Aethoxycarbonyl, n-Propyloxycarbonyl, Isopropyloxycarbonyl oder Isobutyloxycarbonyl, ferner 2-Niederalkoxy-niederalkoxycarbonyl, z.B. 2-Methoxyäthoxycarbonyl, darstellt, als Racematgemische, Racemate oder optische Antipoden.

6. Verbindungen der Formel I, worin R für in 3-, insbesondere aber in 2-Stellung durch Difluormethoxy oder 2-Chlor-1,1,2-trifluoräthoxy, oder für in 3-Stellung, insbesondere aber in 2-Stellung durch Halogenniederalkylthio, z.B. Difluormethylmercapto, oder für in 3-Stellung, insbesondere aber in 2-Stellung durch Niederalkoxyniederalkoxy, z.B. 2-Methoxyäthoxy, oder Niederalkylthioniederalkoxy, z.B. 2-Methylthioäthoxy, substituiertes Phenyl steht, $R_1$ Wasserstoff bedeutet, $R_2$ Niederalkyl, z.B. Methyl ist, und der Rest Ac Niederalkoxycarbonyl, z.B. Methoxycarbonyl, Aethoxycarbonyl oder Isopropyloxycarbonyl, oder Niederalkoxyniederalkoxycarbonyl, z.B. 2-(Methoxy)-äthoxycarbonyl darstellt, als Racematgemische, Racemate oder optische Antipoden.

7. 2-Methyl-4-[2-(2-methoxy-äthoxy)-phenyl]-5-oxo-1,4,5,7-tetrahydrofuro[3,4-b]pyridin-3-carbonsäureäthylester.

8. 2-Methyl-4-[2-(2-methylthio-äthoxy)-phenyl]-5-oxo-1,4,5,7-tetrahydro-furo[3,4,-b]pyridin-3-carbonsäureäthylester.

9. 4-(2-Difluormethoxy-phenyl)-2-methyl-5-oxo-1,4,5,7-tetrahydrofuro[3,4-b]pyridin-3-carbonsäureäthylester.

10. 4-(4-Difluormethoxy-phenyl)-2-methyl-5-oxo-1,4,5,7-tetrahydrofuro[3,4-b]pyridin-3-carbonsäureäthylester.

11. 4-[2-(2-Chlor-1,1,2-trifluor-äthoxy)-phenyl]-2-methyl-5-oxo-1,4,5,7-tetrahydro-furo[3,4-b]pyridin-3-carbonsäureäthylester.

12. 4-(2-Difluormethoxy-phenyl)-2-methyl-5-oxo-1,4,5,7-tetrahydrofuro[3,4-b]pyridin-3-carbonitril.

13. 4-(2-Difluormethoxy-phenyl)-2-methyl-5-oxo-1,4,5,7-tetrahydro-furo-[3,4-b]pyridin-3-carbonsäure-(2-methoxyäthyl)ester.

14. 4-(2-Difluormethoxy-phenyl)-2-methyl-5-oxo-1,4,5,7-tetrahydrofuro[3,4-b]pyridin-3-carbonsäuremethylester.

15. 4-(2-Difluormethoxy-phenyl)-2-methyl-5-oxo-1,4,5,7-tetrahydrofuro[3,4-b]pyridin-3-carbonsäureisopropylester.

16. 4-(3-Difluormethoxy-phenyl)-2-methyl-5-oxo-1,4,5,7-tetrahydro-furo[3,4-b]pyridin-3-carbonsäureäthylester.

17. 4-[2,3-Di-(difluormethoxy)-phenyl]-2-methyl-5-oxo-1,4,5,7-tetrahydro-furo[3,4-b]pyridin-3-carbonsäureäthylester.

18. 4-(2-Difluormethylmercapto-phenyl)-2-methyl-5-oxo-1,4,,5,7-tetrahydro-furo[3,4,-b]pyridin-3-carbonsäureäthylester.

19. Die Verbindungen der Ansprüche 1 - 18 als pharmakologisch aktive, insbesondere Blutdruck-steigernde und cardiotonische, ferner das Zentralnervensystem aktivierende, insbesondere stimulierende sowie als Blutzucker-senkende Verbindungen.

20. Die Verbindungen der Ansprüche 1 - 18 zur Behandlung des menschlichen oder tierischen Körpers.

21. Verwendung der Verbindungen der Ansprüche 1 - 18 zur Behandlung von cardiovaskulären Krankheitszuständen.

22. Verwendung der Verbindungen der Ansprüche 1 - 18 zur Behandlung retardierter Depressionen verschiedenen Schweregrades, von psychischen, von fehlendem Antrieb und Initiative begleiteten Störungen, sowie von hypokinetischen geriatrischen Patienten.

23. Pharmazeutische Präparate enthaltend eine Verbindung der Ansprüche 1 - 18.

24. Verwendung der Verbindungen der Ansprüche 1 - 18 zur Herstellung von pharmazeutischen Präparaten.

25. Verfahren zur Herstellung von pharmazeutischen Präparaten, dadurch gekennzeichnet, dass man Verbindungen der Ansprüche 1 - 18, gegebenenfalls unter Beimischung von Hilfsstoffen, zu pharmazeutischen Präparaten verarbeitet.

26. Verfahren zur Herstellung von Verbindungen der Formel I gemäss Anspruch 1, worin R, $R_1$, $R_2$ und Ac die im Anspruch 1 gegebenen Bedeutungen haben, als Racematgemische, Racemate, optische Antipoden oder Salzen von solchen Verbindungen mit salzbildenden Eigenschaften, dadurch gekennzeichnet, dass man

a) eine Verbindung der Formel

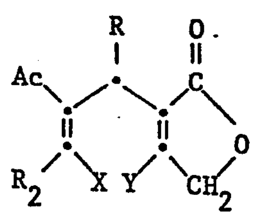

(II)

worin einer der Reste X und Y für die Gruppe der Formel $-NH-R_1$ steht und der andere Hydroxy oder die Gruppe der Formel $-NH-R_1$ bedeutet, oder ein Tautomeres davon oder ein entsprechendes Tautomerengemisch ringschliesst, oder

b) eine Verbindung der Formel

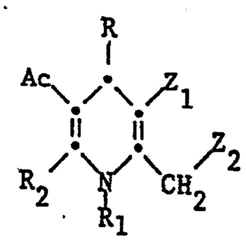

(III)

worin $Z_1$ eine freie, veresterte oder anhydridisierte Carboxylgruppe bedeutet, und $Z_2$ für eine gegebenenfalls reaktionsfähige veresterte Hydroxygruppe steht, ringschliesst, oder

c) eine Verbindung der Formel R-CHO (IV) oder ein reaktionsfähiges funktionelles Derivat davon mit einer Verbindung der Formel

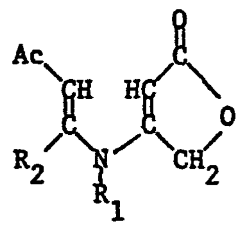

(V)

oder einem Tautomeren davon oder einem entsprechenden Tautomerengemisch umsetzt, oder

d) in einer Verbindung der Formel

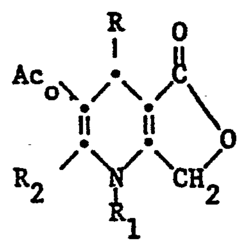

(VI)

worin $Ac_o$ einen in die Gruppe Ac überführbaren Rest darstellt, diesen in letztere überführt, wobei in den obigen Ausgangsstoffen der Formeln II bis VI, die, sofern sie salzbildende Eigenschaften aufweisen, auch in Form ihrer Salze verwendet werden können, die Reste R, $R_1$, $R_2$ und Ac die unter der Formel I gegebenen Bedeutungen haben, und wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die einzelnen Isomeren, oder ein erhaltenes Racemat in die optischen Antipoden auftrennt.

27. Die nach dem Verfahren gemäss Anspruch 26 erhältlichen Verbindungen.